(19) Europäisches Patentamt
European Patent Office
Office européen des brevets

(11) Veröffentlichungsnummer: **0 050 805**
**B1**

(12) # EUROPÄISCHE PATENTSCHRIFT

(45) Veröffentlichungstag der Patentschrift: **24.07.85**

(21) Anmeldenummer: **81108385.6**

(22) Anmeldetag: **15.10.81**

(51) Int. Cl.⁴: **C 07 D 499/00,** C 07 F 9/65, A 61 K 31/43 // (A61K31/43, 31:545)

(54) **Beta-Lactame, Zwischenprodukte und Verfahren zu deren Herstellung, sowie diese enthaltende Arzneimittel.**

(30) Priorität: **24.10.80 CH 7946/80**

(43) Veröffentlichungstag der Anmeldung: **05.05.82 Patentblatt 82/18**

(45) Bekanntmachung des Hinweises auf die Patenterteilung: **24.07.85 Patentblatt 85/30**

(84) Benannte Vertragsstaaten: **AT BE CH DE FR GB IT LI LU NL SE**

(73) Patentinhaber: **F. HOFFMANN-LA ROCHE & CO. Aktiengesellschaft, CH-4002 Basel (CH)**

(72) Erfinder: **Adam-Molina, Solange, Dr., 3 Rue du Montreux, F-68300 St. Louis (FR)**
Erfinder: **Hofheinz, Werner, Dr., Talmattweg 7, CH-4103 Bottmingen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(56) Entgegenhaltungen:
**DE - A - 2 416 492**
**DE - A - 2 708 219**
**GB - A - 1 331 371**
**US - A - 4 123 539**

**THE JOURNAL OF ORGANIC CHEMISTRY, Band 38, Nr. 18, 7. September 1973, Seiten 3227-3228 Department of Chemistry, Massachusetts Institute of Technology Cambridge, Massachusetts J.C. SHEEHAN et al.: "Benzyl 6-oxopenicillanate and derivatives. II"**
**THE JOURNAL OF ORGANIC CHEMISTRY, Band 42, Nr. 24, 25. November 1977, Seiten 3972-3974 Institute of Organic Chemistry, Syntex Research, Palo Alto, California, U.S.A. S. CHANDRASEKARAN et al.: "Synthesis of substituted Beta-lactams by addition of nitromethane to 6-oxopenicillanates and 7-oxocephalosporanates"**
**THE JOURNAL OF ORGANIC CHEMISTRY, Band 40, Nr. 2, 24. Januar 1975, Seiten 191-192 Department of Chemistry, Massachusetts Institute of Technology, Cambridge, Massachusetts, U.S.A. YOUNG S. LO et al.: "6 alpha-Substituted penicillins"**

**EP 0 050 805 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft β-Lactame. Im speziellen betrifft sie β-Lactame der allgemeinen Formel

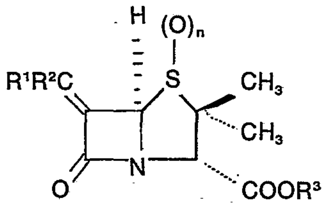

(I)

worin $R^1$ Cyano oder einen Rest der Formel $R^4$–CO– oder $R^5$–A–CO–, $R^2$ Wasserstoff, niederes Alkyl oder Halogen, $R^3$ Wasserstoff, Pivaloyloxymethyl, Acetoxymethyl, 1-Pivaloyloxyäthyl, 1-Acetoxyäthyl, Methoxycarbonyloxymethyl, 1-Äthoxycarbonyloxyäthyl, 1-Isopropoxycarbonyloxyäthyl, Phthalidyl, Thiophthalidyl, Methoxymethyl oder Acetamidomethyl, n die Zahl 0, 1 oder 2, $R^4$ Wasserstoff, Hydroxy, niederes Alkoxy, niederes Alkyl, Aryl oder einen Rest der Formel $R^6ON=C$-$COOR^7$, $R^5$ Halogen, niederes Alkoxy, niederes Alkylthio, niederes Alkylsulfinyl, niederes Alkylsulfonyl, Aryl, Aryloxy, Arylthio, Arylsulfinyl, Arylsulfonyl oder di-(niederes Alkoxy)-phosphinyl, $R^6$ und $R^7$ je niederes Alkyl und A niederes Alkylen bedeuten, wobei «Aryl» einen gegebenenfalls durch Halogen, Nitro, Hydroxy, niederes Alkyl oder niederes Alkoxy substituierten Phenylrest darstellt und «niedere» Gruppen höchstens 7 Kohlenstoffatome tragen,
und pharmazeutisch annehmbare Salze von Carbonsäuren der allgemeinen Formel I mit Basen.

Diese Verbindungen sind neu und zeichnen sich durch therapeutisch wertvolle Eigenschaften aus. Insbesondere besitzen sie ausgeprägte B-Lactamase hemmende Eigenschaften und sind somit nützlich bei der Bekämpfung von β-Lactamase bildenden Krankheitserregern mit β-Lactam-Antibiotika, wie den Penicillinen und Cephalosporinen.

Wenn $R^3$ von Wasserstoff verschieden ist, bedeutet er eine bestimmte, durch Hydrolyse leicht abspaltbare Gruppe, d.h. eine unter neutralen, milden sauren oder milden basischen Bedingungen oder eine enzymatisch, d.h. durch eine Esterase, abspaltbare Gruppe. Es kommen als solche Gruppen in Frage: Pivaloyloxymethyl, Acetoxymethyl, 1-Pivaloyloxyäthyl, 1-Acetoxyäthyl, Methoxycarbonyloxymethyl, 1-Äthoxycarbonyloxyäthyl, 1-Isopropoxycarbonyloxyäthyl, Phthalidyl, Thiophthalidyl, Methoxymethyl und Acetamidomethyl. Der Ausdruck «niederes Alkyl», für sich allein genommen oder in Kombinationen, wie «niederes Alkoxy», «niederes Alkylthio», «niederes Alkylsulfinyl», «niederes Alkylsulfonyl» und dergleichen, bedeutet geradkettige oder verzweigte gesättigte Kohlenwasserstoffreste mit höchstens 7, vorzugsweise höchstens 4, Kohlenstoffatomen, wie Methyl, Äthyl, n-Propyl, Isopropyl, n-Butyl, s-Butyl, t-Butyl und dergleichen. Der Ausdruck «niederes Alkylen» bedeutet geradkettige oder verzweigte gesättigte, zweiwertige Kohlenwasserstoffreste mit höchstens 4 Kohlenstoffatomen, wie Methylen, Äthylen, Äthyliden, 1,2-Propylen und dergleichen. Der Ausdruck «niederes Alkoxy» umfasst Reste, wie Methoxy, Äthoxy, n-Propoxy, Isopropoxy und dergleichen.

Der Ausdruck «niederes Alkylthio» umfasst Reste, wie Methylthio, Äthylthio, n-Propylthio, i-Propylthio und dergleichen. Der Ausdruck «niederes Alkylsulfinyl» umfasst Reste, wie Methylsulfinyl, Äthylsulfinyl, n-Propylsulfinyl, i-Propylsulfinyl und dergleichen. Der Ausdruck «niederes Alkylsulfonyl» umfasst Reste, wie Methylsulfonyl, Äthylsulfonyl, n-Propylsulfonyl, i-Propylsulfonyl und dergleichen. Als Beispiel für einen Rest der Formel $R^6ON=C$–$COOR^7$ sei der Äthoxycarbonyl-methoxyimino-methyl-Rest genannt.

Der Ausdruck «Aryl», für sich allein genommen oder in Kombinationen, wie «Aryloxy», «Arylthio», «Arylsulfinyl», «Arylsulfonyl» und dergleichen, bedeutet einen gegebenenfalls durch Halogen, Nitro, Hydroxy, niederes Alkyl oder niederes Alkoxy substituierten Phenylrest, wie Phenyl, p-Nitrophenyl, p-Bromphenyl, p-Anisyl, o-Hydroxyphenyl, o-Tolyl, p-Tolyl, 2,4,-Dinitrophenyl und dergleichen. Der Ausdruck «Halogen» bedeutet Fluor, Chlor, Brom und Jod.

Der in dieser Beschreibung mehrfach verwendete Ausdruck «pharmazeutisch annehmbare Salze davon mit Basen» bedeutet selbstverständlich pharmazeutisch annehmbare Salze von Carbonsäuren der allgemeinen Formel I mit Basen.

Die Verbindungen der obigen allgemeinen Formel I und pharmazeutisch annehmbare Salze davon mit Basen können, je nach Anordnung der Substituenten $R^1$ bzw. $R^2$, in der (E)- oder in der (Z)-Form oder als Gemische dieser beiden Formen vorliegen. Die vorliegende Erfindung umfasst sowohl die jeweiligen reinen geometrischen Isomeren als auch Mischungen davon.

Gegenstand der vorliegenden Erfindung sind β-Lactame der obigen allgemeinen Formel I und pharmazeutisch annehmbare Salze davon mit Basen als solche und als pharmazeutische Wirkstoffe, die Herstellung dieser Verbindungen und Zwischenprodukte für die Herstellung dieser Verbindungen, ferner Arzneimittel enthaltend eine Verbindung der allgemeinen Formel I oder pharmazeutisch annehmbare Salze davon mit Basen und die Herstellung solcher Arzneimittel.

Unter den Verbindungen der Formel I sind diejenigen bevorzugt, worin $R^1$ Cyano oder einen Rest der Formel $R^4$–CO– oder $R^5$–A–CO–, $R^4$ Wasserstoff, niederes Alkyl, Phenyl oder p-Nitrophenyl, $R^5$ Chlor, Methylthio, Methylsulfonyl, Phenyl, Phenoxy, Phenylthio oder Phenylsulfonyl und A Methylen bedeuten. Die bevorzugte Bedeutungsmöglichkeit von $R^2$ ist Wasserstoff oder Halogen. $R^3$ bedeutet vorzugsweise Wasserstoff, Acetoxymethyl oder Pivaloyloxymethyl. Die bevorzugte Bedeutungsmöglichkeit von n ist die Zahl 0.

Ganz besonders bevorzugte Verbindungen im Rahmen der vorliegenden Erfindung sind:

Methylen-(2S,5R)-6-[(Z)-acetonyliden]-3,3-dimethyl-7-oxo-4-thia-1-azabicyclo[3.2.0]heptan-2-carboxylat-pivalat und
Natrium-(2S,5R)-6-[(Z)-acetonyliden]-3,3-dimethyl-7-oxo-4-thia-1-azabicyclo[3.2.0]heptan-2-carboxylat.

Weitere besonders bevorzugte Verbindungen im Rahmen der vorliegenden Erfindung sind:
Methylen-(2S,5R)-3,3-dimethyl-6-(3-methyl-2-oxobutyliden)-7-oxo-4-thia-1-azabicyclo[3.2.0]heptan-2-carboxylat-pivalat,
Methylen-(2S,5R)-3,3-dimethyl-7-oxo-6-(2-oxo-3-phenoxypropyliden)-4-thia-1-azabicyclo[3.2.0]heptan-2-carboxylat-pivalat,
Methylen-(2S,5R)-3,3-dimethyl-7-oxo-6-(2-oxo-3-phenylpropyliden)-4-thia-1-azabicyclo[3.2.0]heptan-2-carboxylat-pivalat und
Methylen-(2S,5R)-3,3-dimethyl-7-oxo-6-[3-methylthio-2-oxopropyliden]-4-thia-1-azabicyclo[3.2.0]heptan-2-carboxylat-pivalat.

Weitere bevorzugte Verbindungen im Rahmen der vorliegenden Erfindung sind:
Methylen-(2S,5R)-6-acetonyliden-3,3-dimethyl-7-oxo-4-thia-1-azabicyclo[3.2.0]heptan-2-carboxylat-pivalat-4,4-dioxid,
Methylen-(2S,5R)-3,3-dimethyl-6-(1-chlor-2-oxopropyliden)-7-oxo-4-thia-1-azabicyclo-[3.2.0]heptan-2-carboxylat-pivalat,
Methylen-(2S,5R)-6-(3-chloracetonyliden)-3,3-dimethyl-7-oxo-4-thia-1-azabicyclo[3.2.0]heptan-2-carboxylat-pivalat,
Methylen-(2S,5R)-6-acetonyliden-3,3-dimethyl-7-oxo-4-thia-1-azabicyclo[3.2.0]heptan-2-carboxylat-pivalat-4-oxid und
(E)- und (Z)-Methylen(2S,5R)-6-(formylmethylen)-3,3-dimethyl-7-oxo-4-thia-1-azabicyclo-[3.2.0]heptan-2-carboxylat-pivalat.

Die Verbindungen der allgemeinen Formel I und pharmazeutisch annehmbare Salze davon mit Basen können erfindungsgemäss hergestellt werden, indem man

a) eine Verbindung der allgemeinen Formel

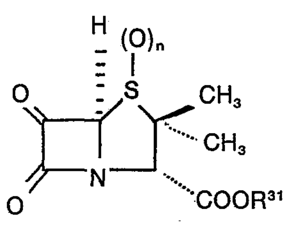

(II)

worin $R^{31}$ eine durch Hydrolyse leicht abspaltbare Gruppe bedeutet, und n obige Bedeutung besitzt,
mit einem Phosphoran der allgemeinen Formel

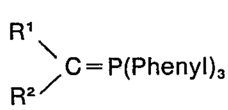

(III)

worin $R^1$ und $R^2$ obige Bedeutung besitzen, umsetzt, oder

b) eine Verbindung der allgemeinen Formel

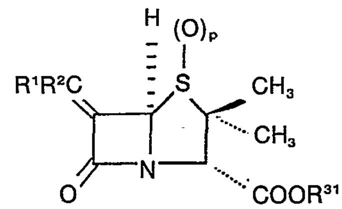

(Ia)

worin p die Zahl 0 oder 1 bedeutet, und $R^1$, $R^2$ und $R^{31}$ obige Bedeutung besitzen,
am (an den) Schwefelatom(en) oxidiert, oder

c) in einer Verbindung der allgemeinen Formel

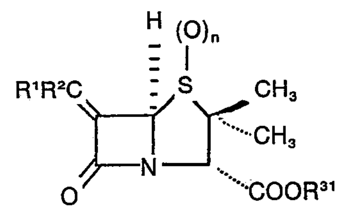

(Ib)

worin $R^1$, $R^2$, $R^{31}$ und n obige Bedeutung besitzen, die Estergruppe hydrolysiert,

d) erwünschtenfalls ein erhaltenes Gemisch der (E)- und (Z)-Isomeren auftrennt, und

e) erwünschtenfalls eine erhaltene Carbonsäure der allgemeinen Formel I mit einer Base in ein pharmazeutisch annehmbares Salz überführt.

Gemäss Verfahrensvariante a) können Verbindungen der allgemeinen Formel I, worin $R^3$ eine durch Hydrolyse leicht abspaltbare Gruppe bedeutet, und $R^1$, $R^2$ und n die eingangs erwähnte Bedeutung besitzen, hergestellt werden, indem man eine Carbonylverbindung der allgemeinen Formel II in an sich bekannter Weise mit einem Phosphoran der allgemeinen Formel III umsetzt. Diese Reaktion wird zweckmässigerweise in einem unter den Reaktionsbedingungen inerten organischen Lösungsmittel durchgeführt, wobei in erster Linie Kohlenwasserstoffe, wie Benzol, Toluol, Xylol, Pentan und Hexan, halogenierte Kohlenwasserstoffe, wie Methylenchlorid, Chloroform, Tetrachlorkohlenstoff, 1,2-Dichloräthan und Chlorbenzol, und Äther, wie t-Butyl-methyläther, Tetrahydrofuran, Dioxan und Diäthyläther, in Frage kommen. Zweckmässigerweise arbeitet man in einem Temperaturbereich von etwa 0 °C bis etwa 50 °C, vorzugsweise bei Raumtemperatur.

Die Verbindung der Formel II muss dabei nicht notwendigerweise in isoliertem Zustand eingesetzt werden; man kann das Reaktionsgemisch, in dem sie hergestellt worden ist, direkt in einem sogenannten «Eintopfverfahren» weiterverarbeiten.

Gemäss Verfahrensvariante b) können Verbindungen der allgemeinen Formel I, worin n nicht die Zahl 0 bedeutet, hergestellt werden, indem man eine Verbindung der allgemeinen Formel Ia

am Schwefelatom oxidiert, wobei eine allenfalls im Rest $R^1$ (in der Bedeutung $R^5$–A–CO–) vorhandene Thio- oder Sulfinylgruppe ebenfalls oxidiert werden kann. Diese Reaktion kann mit jedem geeigneten Oxidationsmittel nach an sich bekannten Methoden durchgeführt werden. Für den vorliegenden Verfahrensaspekt geeignete Oxidationsmittel umfassen Peroxide, wie Wasserstoffperoxid, t-Butylhydroperoxid und dergleichen, und Persäuren, wie Perameisensäure, Peressigsäure, Perbenzoesäure, m-Chlorperbenzoesäure, Perjodsäure, Kalium- oder Natriummetaperjodat, und dergleichen. Geeignete Lösungsmittel sind, je nach verwendetem Oxidationsmittel, Kohlenwasserstoffe, wie Benzol und Toluol, halogenierte Kohlenwasserstoffe, wie Dichlormethan, Chloroform und 1,2-Dichloräthan, niedere Fettsäuren, wie Ameisensäure, Essigsäure, und dergleichen. Je nach Reaktivität der verwendeten Ausgangsstoffe und Oxidationsmittel kann die Reaktionstemperatur in einem Bereich von etwa 0 °C bis Siedetemperatur der Reaktionsmischung variieren.

Je nach eingesetztem Oxidationsmittel und/oder Variation der Reaktionsbedingungen, ist es ohne weiteres möglich eine Verbindung der allgemeinen Formel Ia, worin n die Zahl 0 bedeutet, zu einer Verbindung der allgemeinen Formel Ia, worin n die Zahl 1 bedeutet zu oxidieren. Die Oxidation eines Sulfids der allgemeinen Formel Ia zum entsprechenden Sulfon kann demnach in einem Schritt oder in zwei Schritten erfolgen. Beispielsweise erhält man bei der Oxidation eines Sulfids der allgemeinen Formel Ia mit etwa zwei Äquivalenten m-Chlorperbenzoesäure in siedendem Dichlormethan direkt das entsprechende Sulfon; arbeitet man hingegen bei Raumtemperatur und verwendet etwa ein Äquivalent m-Chlorperbenzoesäure so erhält man das entsprechende Sulfoxid, das dann erwünschtenfalls zum entsprechenden Sulfon weiteroxidiert werden kann. Die Wahl des geeigneten Oxidationsmittels und der geeigneten Reaktionsbedingungen bietet dem Fachmann keinerlei Schwierigkeiten.

Gemäss Verfahrensvariante c) kann eine Carbonsäure der allgemeinen Formel I, d.h. eine Verbindung der allgemeinen Formel I, worin $R^3$ Wasserstoff bedeutet, hergestellt werden, indem man in einer Verbindung der allgemeinen Formel Ib die Estergruppe hydrolysiert. Diese Hydrolyse kann nach an sich bekannten und in solchen Fällen üblichen Methoden durchgeführt werden, wobei die Wahl der geeigneten Methode und der geeigneten Reaktionsbedingungen dem Fachmann keinerlei Schwierigkeiten bereiten.

In einer bevorzugten Ausführungsform wird die Hydrolyse einer Verbindung der allgemeinen Formel Ib enzymatisch mit einer Esterase, wie beispielsweise Schweineleber-Esterase, durchgeführt, und zwar in einem bei solchen Reaktionen üblichen wässrigen, gepufferten System, beispielsweise in Phosphat-Puffer (pH 7), wobei gegebenenfalls Lösungsvermittler, wie Dimethylsulfoxid oder dergleichen, zugesetzt werden können. Die Reaktionstemperatur liegt, wie in solchen Fällen üblich, in einem Bereich von etwa 20 °C bis 40 °C.

Gemäss Verfahrensvariante d) kann man ein erhaltenes Gemisch der (E)- und (Z)-Isomeren von Verbindungen der allgemeinen Formel I auftrennen. Diese Auftrennung erfolgt nach an sich bekannten und jedem Fachmann geläufigen Methoden. Eine für den vorliegenden Verfahrensaspekt besonders geeignete Methode stellt die Chromatographie dar.

Gemäss Verfahrensvariante e) kann eine Carbonsäure der allgemeinen Formel I mit einer Base in ein pharmazeutisch annehmbares Salz übergeführt werden. Geeignete Basen sind beispielsweise anorganische Base, wie Natriumhydroxid, Natriumcarbonat, Natriumbicarbonat, entsprechende Kalium- oder Kalziumsalze oder dergleichen, und organische Basen, wie Triäthylamin, Piperidin, Diäthylamin oder dergleichen. Solche Salze können im Hinblick auf den Stand der Technik und die vorliegenden Verbindungen von jedem Fachmann leicht hergestellt werden.

Die als Ausgangsstoffe verwendeten Verbindungen der allgemeinen Formel II können beispielsweise aus der Carbonsäure der Formel

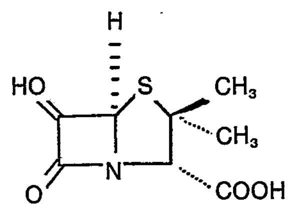

(IV)

leicht hergestellt werden. In einem ersten Schritt kann man die Carbonsäure der Formel IV, beispielsweise durch Behandlung eines Salzes davon, (z.B. des Natrium- oder Kaliumsalzes) mit einem geeigneten die Gruppe $R^{31}$ liefernden Mittel, wie Pivaloyloxymethyljodid, Acetoxymethyljodid oder dergleichen, in einem inerten organischen Lösungsmittel, wie Dimethylformamid, Dimethylacetamid, Dimethylsulfoxid, Hexamethylphosphorsäuretriamid in eine Verbindung der allgemeinen Formel

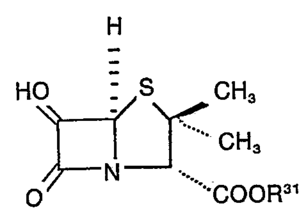

(V)

worin $R^{31}$ obige Bedeutung besitzt,

überführen. Durch Oxidation der sekundären Hydroxylgruppe in einer Verbindung der allgemeinen Formel V, beispielsweise mit Trifluoressigsäureanhydrid und Dimethylsulfoxid, in einem inerten organischen Lösungsmittel, vorzugsweise in einem halogenierten Kohlenwasserstoff, wie Dichlormethan, Chloroform und dergleichen, erhält man eine Verbindung der allgemeinen Formel II, worin n die Zahl 0 bedeutet. Eine so erhaltene

Verbindung kann anschliessend in Analogie zu Verfahrensvariante b), oxidiert werden, wobei man Verbindungen der allgemeinen Formel II, worin n die Zahl 1 oder 2 bedeutet, erhält.

Wie bereits eingangs erwähnt zeigen die erfindungsgemässen Verbindungen der allgemeinen Formel I und pharmazeutisch annehmbare Salze davon mit Basen ausgeprägte β-Lactamase hemmende Aktivitäten gegen β-Lactamasen aus verschiedenen Bakterienstämmen. Diese therapeutisch wertvollen Eigenschaften können, wie nachstehend erläutert, an isolierten β-Lactamasen in vitro bestimmt werden:

A. Isolierung der β-Lactamasen

Verschiedene β-Lactamasen können aus penicillin- bzw. cephalosporin-resistenten Bakterienstämmen, wie Klebsiella pneumoniae NCTC 418, Proteus vulgaris 1028 und Escherichia coli 1024, isoliert werden. Hierzu werden die entsprechenden Stämme in Tryptic Soy Broth (Difco) kultiviert und durch Zentrifugation in der späten logarithmischen Wachstumsphase geerntet (wenn nötig gibt man dem Medium gegen Ende der log-Phase 50–100 mg/l Ampicillin hinzu, um die β-Lactamase zu induzieren). Die so erhaltene Bakterienmasse wird mit 10 mM Phosphatpuffer (pH 7,0) versetzt; unter Kühlung werden die Zellen mit Ultraschall (Biosonik III, Bronwill; 3–5 min-Impulse) aufgebrochen. Man zentrifugiert (20 000 U/min.) während 20–30 Minuten und erhält einen klaren Rohextrakt, den man als Enzymquelle (β-Lactamase-Quelle) verwenden kann, und der ohne Aktivitätsverlust über mehrere Monate bei −20 °C eingefroren werden kann.

B. Bestimmung der β-Lactamase-Aktivität

Die Bestimmung der Aktivität der isolierten β-Lactamasen kann nach der Methode von O'Callaghan, C.H. et al. [Antimicr. Ag. Chemother. 1, 283–288 (1972)] mit dem chromogenen Cephalosporin Nitrocefin (87/312 von Glaxo) durchgeführt werden. Der benötigte Versuchsansatz enthält pro ml Wasser: 50 mM Phosphatpuffer (pH 7,0), 0,1 mM Nicrocefin und genügend Enzym (β-Lactamase) um eine ΔA/min. von ca. 0,1 zu erreichen. Die Spaltung des Substrates, die mit einer Farbänderung verbunden ist, erfolgt bei 37 °C und wird bei 482 nm mit einem Spektralphotometer quantitativ verfolgt.

C. Bestimmung der β-Lactamase hemmenden Wirkung der Verbindungen der allgemeinen Formel I

Die oben beschriebene Spaltung des chromogenen Substrates durch β-Lactamasen (Versuch B.) kann durch Zugabe von Verbindungen der allgemeinen Formel I (Inhibitoren) gehemmt werden. Da es sich zeigte, dass die Inhibitoren die β-Lactamase in einer zeitabhängigen Reaktion irreversibel inaktivieren, wird die Reaktion (Spaltung des Substrates), jeweils nach einer Vorinkubationszeit von β-Lactamase mit Inhibitor von 15 Minuten, durch Zugabe des Substrates gestartet. Die Bestimmung der β-Lactamase hemmenden Aktivität von Verbindungen der allgemeinen Formel I, worin $R^1$ eine durch Hydrolyse leicht abspaltbare Gruppe bedeutet, erfolgte jeweils in Gegenwart und Abwesenheit einer Esterase. Als Mass für die Affinität des jeweils getesteten Inhibitors zur β-Lactamase, die ein Mass für die Stärke des Inhibitors darstellt, dient diejenige Konzentration, welche die unter obigen Versuchsbedingungen (Versuch B.) in Abwesenheit eines Inhibitors erfolgende Spaltung des Substrates (Nitrocefin) zu 50% hemmt (IC 50 in μM/l). Zur Bestimmung der IC 50 wurden 4 bis 6 Versuche mit verschiedenen Konzentrationen an Inhibitor durchgeführt. Die Ermittlung der IC 50 erfolgte graphisch.

Die in obigem Versuch (Versuch C.) erhaltenen Resultate sind in der nachfolgenden Tabelle dargestellt:

Tabelle 1

| erfindungsgemässe Produkte | | | | β-Lactamase-Hemmung: IC 50 in μM/l | | | | | |
|---|---|---|---|---|---|---|---|---|---|
| $R^1$ | $R^2$ | $R^3$ | n | PV 1028 | | KP NCTC 418 | | E.c. 1024 | |
| | | | | −E | +E | −E | +E | −E | +E |
| $CH_3CO-$ | H | Piv | 0 | 0,16 | 0,015 | 0,19 | 0,005 | 32 | 1,5 |
| $CH_3CO-$ | H | H | 0 | 0,0039 | | 0,0046 | | 1,65 | |
| $CH_3CO-$ | H | Piv | 1 | 0,76 | 0,16 | 28 | 1,3 | >100 | 28 |
| $CH_3CO-$ | H | Piv | 2 | 1,3 | 0,51 | 1,9 | 0,18 | 18 | 1,6 |
| Ø-$OCH_2CO-$ | H | H | 0 | – | | 0,085 | | 0,75 | |
| NC- | H | H | 0 | 0,095 | | 14 | | 6,5 | |
| $(CH_3)_2CHCO-$ | H | Piv | 0 | 0,25 | 0,0022 | 2,3 | 0,18 | 3,5 | 0,7 |
| Ø-$OCH_2CO-$ | H | Piv | 0 | 0,7 | 0,011 | 3,5 | 0,06 | 29 | – |
| Ø-$CH_2CO-$ | H | Piv | 0 | 0,14 | 0,011 | 0,07 | 0,0029 | >100 | 2,8 |
| $CH_3-S-CH_2CO-$ | H | Piv | 0 | 0,6 | 0,002 | 3 | 0,065 | 55 | 3,0 |

−E = in Abwesenheit einer Esterase
+E = in Gegenwart einer Esterase
Ø = Phenyl
Piv = Pivaloyloxymethyl

Tabelle 1 Fortsetzung

| erfindungsgemässe Produkte | | | | β-Lactamase-Hemmung: IC 50 in µM/l | | | | | |
|---|---|---|---|---|---|---|---|---|---|
| $R^1$ | $R^2$ | $R^3$ | n | PV 1028 | | KP NCTC 418 | | E.c. 1024 | |
| | | | | −E | +E | −E | +E | −E | +E |
| Ø-$SO_2$-$CH_2CO$- | H | Piv | 0 | 3,5 | 0,1 | 40 | 5 | 52 | 11,5 |
| $CH_3CO$- | Cl | Piv | 0 | 0,9 | 0,62 | 4,6 | 0,22 | 4 | 0,4 |
| Cl-$CH_2CO$- | H | Piv | 0 | 0,01 | 0,032 | 7,6 | 0,46 | >100 | 8 |
| HCO- | H | Piv | 0 | 4,9 | 0,0045 | 12 | 0,7 | >100 | 16 |
| $(EtO)_2PO$-$CH_2CO$- | H | Piv | 0 | 0,055 | 0,01 | 9,4 | 1,3 | >100 | 16 |
| $CH_3$-$SO_2$-$CH_2CO$- | H | Piv | 0 | 3,1 | 0,9 | 100 | 5,2 | >100 | 20 |
| p-$NO_2$-Ø-CO- | H | Piv | 0 | 0,53 | 0,044 | 100 | 10 | 85 | 5,5 |

In DE-A-2 416 492 und J. Org. Chem. Bd. 38, No. 18, 1973, Seiten 3227–8 werden Benzylester von β-Lactamen mit ähnlicher Substitution in 6-Stellung wie die erfindungsgemässen Produkte offenbart. Demgegenüber sind die erfindungsgemässen Verbindungen der Formel I entweder freie Carbonsäuren oder auch in 3-Stellung mit einer durch Hydrolyse, d.h. unter neutralen, milden sauren oder milden basischen Bedingungen oder insbesondere durch die herkömmlichen Esterasen im Menschen, Affen und Hund abspaltbaren, bestimmten Gruppe verestert. Die vorbekannten Benzylester zählen nicht zu diesen leicht abspaltbaren Estern und sind bezeichnenderweise auch praktisch ohne β-Lactamase hemmende Wirkung. Auch die weiteren, in DE-A-2 416 492 aufgeführten Ester sind anderer Natur als die erfindungsgemässen, leicht abspaltbaren Ester. Die neuen β-Lactame der Formel I sowie die entsprechenden pharmazeutisch annehmbaren Salze von Carbonsäuren der Formel I stellen deshalb, im Hinblick auf die gefundene β-Lactamase hemmende Wirkung, eine wertvolle Bereicherung in der Chemotherapie von Infektionskrankheiten dar.

Die Verbindungen der allgemeinen Formel I und pharmazeutisch annehmbare Salze davon mit Basen können z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z.B. Lactose, Maisstärke oder Derivate davon, Talk, Stearinsäure oder deren Salze etc. verwenden. Für Weichgelatinekapseln eignen sich als Träger z.B. pflanzliche Öle, Wachse, Fette, halbfeste und flüssige Polyole etc. Zur Herstellung von Lösungen und Sirupen eignen sich als Träger z.B. Wasser, Polyole, Saccharose, Invertzucker, Glukose und dergleichen. Für Injektionslösungen eignen sich als Träger z.B. Wasser, Alkohole, Polyole, Glyzerin, pflanzliche Öle etc. Für Suppositorien eignen sich als Träger z.B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Die Carbonsäuren der allgemeinen Formel I und ihre pharmazeutisch annehmbaren Salze mit Basen kommen vorzugsweise für die parenterale Applikation in Betracht und werden zu diesem Zweck bevorzugt als Lyophilisate oder Trockenpulver zubereitet. Die leicht hydrolysierbaren Ester der allgemeinen Formel I kommen vorzugsweise für die enterale Applikation in Betracht.

Wie eingangs erwähnt, kann man Verbindungen der allgemeinen Formel I und pharmazeutisch annehmbare Salze davon mit Basen erfindungsgemäss bei der Bekämpfung bzw. Verhütung von Krankheiten verwenden, insbesondere bei der Bekämpfung von β-Lactamase bildenden Krankheitserregern in Kombination mit β-Lactam-Antibiotika, d.h. Antibiotika welche einen β-Lactamring enthalten, beispielsweise Penicilline, wie Benzylpenicillin, Phenoxymethylpenicillin, Carbenicillin, Methicillin, Propicillin, Ampicillin, Amoxicillin und Mecillinam, und Cephalosporine, wie Cephaloridin, Cephalotin, Cefazolin, Cephalexin, Cefoxitin, Cephacetril, Cephamandol, Cephapirin, Cephradin und Cephaloglycin. Dabei können die Verbindungen der allgemeinen Formel I oder pharmazeutisch annehmbaren Salze davon mit Basen vor, gleichzeitig mit oder nach der Verabreichung bzw. Einnahme von β-Lactam-Antibiotika verabreicht werden. Werden die erfindungsgemässen Produkte gleichzeitig mit einem β-Lactam-Antibiotikum verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Salz davon mit Base und ein β-Lactam-Antibiotikum enthält; derartige pharmazeutische Kombinationen sind ebenfalls Gegenstand der vorliegenden Erfindung.

Die Dosierung der Verbindungen der allgemeinen Formel I und der pharmazeutisch annehmbaren Salze davon mit Basen kann innerhalb weiter Grenzen variieren und ist natürlich in jedem einzelnen Fall den individuellen Gegebenheiten und dem zu bekämpfenden ß-Lactamase produzierenden Krankheitserreger anzupassen. Im allgemeinen dürfte eine Tagesdosis von etwa 0,1 bis etwa 2,0 g angemessen sein. Das Verhältnis von β-Lactamase-Inhibitor (Verbindung der Formel I oder pharmazeutisch annehmbares Salz davon mit Base) zu β-Lactam-Antibiotikum kann ebenfalls innerhalb weiter Grenzen variieren und ist in jedem einzelnen Fall den individuellen Gegebenheiten anzupassen. Im allgemeinen dürfte ein Verhältnis von etwa 1 : 20 bis etwa 1 : 1 angemessen sein.

Wie eingangs erwähnt, sind Arzneimittel, enthaltend eine Verbindung der allgemeinen Formel I oder ein pharmazeutisch annehmbares Salz davon mit Base, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch annehmbare Salze davon mit Basen und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Insbesondere sind pharmazeutische Kombinationen, enthaltend eine Verbindung der allgemeinen Formel I oder ein pharmazeutisch annehmbares Salz davon mit Base und ein β-Lactam-Antibiotikum, z.B. ein Penicillin, wie Benzylpenicillin, Phenoxymethylpenicillin, Carbenicillin, Methicillin, Propicillin, Ampicillin, Amoxicillin und Mecillinam, oder ein Cephalosporin, wie Cephaloridin, Cephalotin, Cefazolin, Cephalexin, Cefoxitin, Cephacetril, Cephamandol, Cephapirin, Cephradin und Cephaloglycin, Gegenstand der vorliegenden Erfindung. Derartige Kombinationen eignen sich zur Bekämpfung von β-Lactamase bildenden Krankheitserregern.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) Zu einer eisgekühlten Lösung von 20 g (2S,5R,6S)-Hydroxy-7-oxo-3,3-dimethyl-4-thia-1-azabicyclo[3.2.0]heptan-2-carbonsäure-Kaliumsalz in 50 ml Dimethylformamid tropft man unter Rühren 20 g Pivaloyloxymethyl-jodid hinzu. Nach 3 Stunden giesst man auf 200 g Eis, und extrahiert das Gemisch mit 1000 ml Äther. Die organische Phase wird neutral gewaschen, über Magnesiumsulfat getrocknet, filtriert und eingedampft. Der ölige Rückstand wird unter Eluieren mit Cyclohexan/Essigester (6 : 4) an Kieselgel chromatographiert. Man erhält Methylen-(2S,5R,6S)-hydroxy-7-oxo-3,3-dimethyl-4-thia-1-azabicyclo[3.2.0]heptan-2-carboxylat-pivalat [Rf: 0,21; Cyclohexan/Essigester (6 : 4); NMR ($CDCl_3$), δ (ppm): 1,24 (9H); 1,48 (3H); 1,6 (3H); 4,5 (1H); 4,84 (1H); 5,3 (1H); 5,85 (2H)].

b) Man löst 8,9 ml Dimethylsulfoxid in 150 ml Dichlormethan, und versetzt die erhaltene Lösung tropfenweise bei −65° unter Rühren mit einer Lösung von 10,5 ml Trifluoressigsäureanhydrid in 100 ml Dichlormethan. Nach beendeter Zugabe wird sodann eine Lösung von 20,8 g Methylen-(2S,5R,6S)-hydroxy-7-oxo-3,3-dimethyl-4-thia-1-azabicyclo[3.2.0]heptan-2-carboxylat-pivalat in 150 ml Dichlormethan zugetropft. Die erhaltene gelbe Lösung wird anschliessend während 30 Minuten bei −65° gerührt und dann tropfenweise mit 20 ml Triäthylamin versetzt. Man lässt das Reaktionsgemisch bei Raumtemperatur stehen, giesst auf Eis und extrahiert mit Dichlormethan. Die vereinigten organischen Phasen werden mit Natriumbicarbonatlösung extrahiert, neutral gewaschen, über Magnesiumsulfat getrocknet, filtriert und eingedampft. Man erhält Methylen-6,7-dioxo-3,3-dimethyl-4-thia-1-azabicyclo[3.2.0]heptan-2-carboxylat-pivalat als öligen Rückstand [Rf: 0,17; Cyclohexan/Essigester (6 : 4); NMR ($CDCl_3$), δ (ppm): 1,24 (9H); 1,53 (6H); 4,8 ($1H_3$); 5,8 ($1H_5$); 5,83 (2H)].

c) Eine Lösung von 3,5 g Methylen-6,7-dioxo-3,3-dimethyl-4-thia-1-azabicyclo[3.2.0]heptan-2-carboxylat-pivalat in 150 ml Benzol wird bei Raumtemperatur mit 6,3 g 1-Triphenylphosphoranyliden-2-propanon versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-6-[(E)-acetonyliden]-3,3-dimethyl-7-oxo-4-thia-1-azabicyclo[3.2.0]heptan-2-carboxylat-pivalat [Rf: 0,47; Cyclohexan/Essigester (6 : 4); NMR ($CDCl_3$), δ (ppm): 1,24 (9H); 1,52 (3H); 1,62 (3H); 2,62 (3H, Ac); 4,65 ($1H_3$); 5,82 ($1H_5$); 5,9 (m, 2H); 6,11 (1H)] und Methylen-(2S,5R)-6-[(Z)-acetonyliden]-3,3-dimethyl-7-oxo-4-thia-1-azabicyclo[3.2.0]heptan-2-carboxylat-pivalat [Rf: 0,53; Cyclohexan/Essigester (6 : 4); NMR ($CDCl_3$), δ (ppm): 1,25 (9H); 1,53 (3H); 1,61 (3H); 2,38 (3H, Ac); 4,64 ($1H_3$); 5,91 (m, 2H); 6,08 ($1H_5$); 6,63 (1H)].

## Beispiel 2

Eine Lösung von 3,7 g Methylen-6,7-dioxo-3,3-dimethyl-4-thia-1-azabicyclo[3.2.0]heptan-2-carboxylat-pivalat in 100 ml Benzol wird bei Raumtemperatur mit 4,7 g Phenoxyacetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-7-oxo-6-(2-oxo-3-phenoxypropyliden)-4-thia-1-azabicyclo[3.2.0]heptan-2-carboxylat-pivalat als gelbes Öl [Rf: 0,44; Cyclohexan/Essigester (6 : 4); NMR ($CDCl_3$), δ (ppm): 1,21 (9H); 1,51 (3H); 1,56 (3H); 4,58 (1H); 5,66 (2H); 5,83 (2H); 6,05 (1H); 6,8–7,45 (6H)].

Beispiel 3

Eine Lösung von 3,2 g Methylen-6,7-dioxo-3,3-dimethyl-4-thia-1-azabicyclo[3.2.0]heptan-2-carboxylat-pivalat in 150 ml Benzol wird bei Raumtemperatur mit 5 g Cyanomethylentriphenylphosphoran versetzt. Nach 10 Minuten wird das Reaktionsgemisch eingedampft. Der Rückstand wird unter Eluieren mit Cyclohexan/Essigester (7 : 3) an Kieselgel chromatographiert. Man erhält Methylen-(2S,5R)-6-[(Z)-cyanomethylen]-3,3-dimethyl-7-oxo-4-thia-1-azabicyclo[3.2.0]heptan-2-carboxylat-pivalat als rötliches Öl [Rf: 0,38; Cyclohexan/Essigester (6 : 4); NMR ($CDCl_3$), δ (ppm): 1,24 (9H); 1,53 (3H); 1,59 (3H); 4,59 (1H); 5,84 (2H); 5,89 (1H); 5,97 (1H)] und Methylen-(2S,5R)-6-[(E)-cyanomethylen]-3,3-dimethyl-7-oxo-4-thia-1-azabicyclo[3.2.0]heptan-2-carboxylat-pivalat als rötliches Öl [Rf: 0,33; Cyclohexan/Essigester (6 : 4); NMR ($CDCl_3$), δ (ppm): 1,24 (9H); 1,51 (3H); 1,6 (3H); 4,64 (1H); 5,59 (1H); 5,84 (3H)].

Beispiel 4

Eine Lösung von 7,8 g Methylen-6,7-dioxo-3,3-dimethyl-4-thia-1-azabicyclo[3.2.0]heptan-2-carboxylat-pivalat in 200 ml Benzol wird bei Raumtemperatur mit 10 g Triphenylphosphinmethylacetylmethylen versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-6-(1-methyl-2-oxopropyliden)-7-oxo-4-thia-1-azabicyclo-[3.2.0]heptan-2-carboxylat-pivalat als weisse Kristalle vom Schmelzpunkt 59–60° [Rf: 0,30; Cyclohexan/Essigester (6 : 4)].

Beispiel 5

Eine Lösung von 5,4 g Methylen-6,7-dioxo-3,3-dimethyl-4-thia-1-azabicyclo[3.2.0]heptan-2-carboxylat-pivalat in 100 ml Benzol wird bei Raumtemperatur mit 9,6 g Phenylacetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-7-oxo-6-(2-oxo-3-phenylpropyliden)-4-thia-1-azabicyclo-[3.2.0]heptan-2-carboxylat-pivalat als gelbes Öl [Rf: 0,4; Cyclohexan/Essigester (6 : 4); NMR ($CDCl_3$), δ (ppm): 1,23 (9H); 1,5 (3H); 1,56 (3H); 3,88 (2H); 4,58 (1H); 5,88 (2H); 6,05 (1H); 6,63 (1H); 7,37 (m, 5H)].

Beispiel 6

Eine Lösung von 5,4 g Methylen-6,7-dioxo-3,3-dimethyl-4-thia-1-azabicyclo[3.2.0]heptan-2-carboxylat-pivalat in 100 ml Benzol wird bei Raumtemperatur mit 8,8 g Dimethylacetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-6-(3-methyl-2-oxobutyliden)-7-oxo-4-thia-1-azabicyclo-[3.2.0]heptan-2-carboxylat-pivalat als gelbes Öl [Rf: 0,35; Cyclohexan/Essigester (6 : 4); NMR ($CDCl_3$), δ (ppm): 1,12 (6H); 1,25 (9H); 1,51 (3H); 1,6 (3H); 2,77 (2H); 4,6 (1H); 5,89 (2H); 6,05 (1H); 6,72 (1H)].

Beispiel 7

Eine Lösung von 2,1 g Methylen-6,7-dioxo-3,3-dimethyl-4-thia-1-azabicyclo[3.2.0]heptan-2-carboxylat-pivalat in 100 ml Benzol wird bei Raumtemperatur mit 2,7 g Triphenylphosphinchloracetylmethylen versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-6-(1-chlor-2-oxo-propyliden)-7-oxo-4-thia-1-azabicyclo-[3.2.0]heptan-2-carboxylat-pivalat als Öl [Rf: 0,45; Cyclohexan/Essigester (6 : 4); NMR ($CDCl_3$), δ (ppm): 1,25 (9H); 1,51 (3H); 1,6 (3H); 2,53 und 2,74 (3H); 4,66 (1H); 5,9 (2H); 6,08 (1H)].

Beispiel 8

Eine Lösung von 3,3 g Methylen-6,7-dioxo-3,3-dimethyl-4-thia-1-azabicyclo[3.2.0]heptan-2-carboxylat-pivalat in 100 ml Benzol wird bei Raumtemperatur mit 6 g Phenylsulfonylacetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-7-oxo-6-[3-(phenylsulfonyl)-2-oxopropyliden]-4-thia-1-azabicyclo[3.2.0]heptan-2-carboxylat-pivalat als gelbe Kristalle vom Schmelzpunkt 100–102° [Rf: 0,25; Cyclohexan/Essigester (6 : 4)].

Beispiel 9

Eine Lösung von 4,7 g Methylen-6,7-dioxo-3,3-dimethyl-4-thia-1-azabicyclo[3.2.0]heptan-2-carboxylat-pivalat in 150 ml Benzol wird bei Raumtemperatur mit 9 g p-Nitrobenzoyltriphenylmethylenphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-6-(p-nitrophenacyliden)-7-oxo-4-thia-1-azabicyclo-[3.2.0]heptan-2-carboxylat-pivalat als orange Kristalle vom Schmelzpunkt 96–98°.

Beispiel 10

Eine Suspension von 1,7 g Methylen-(2S,5R)-6-acetonyliden-3,3-dimethyl-7-oxo-4-thia-1-azabicyclo[3.2.0]heptan-2-carboxylat-pivalat in 25 ml Dimethylsulfoxid und 500 ml Phosphat-Puffer (pH 7) wird bei 20° mit 1,7 ml einer Suspension von Schweineleber-Esterase (3 mg/ml) versetzt. Man rührt während 3 Stunden bei 20° und extrahiert anschliessend das Reaktionsgemisch mit Äther. Die wässrige Phase wird mit 50-proz. Citronensäure angesäuert (pH 3,2) und mit Äther ausgeschüttelt. Der organische Auszug wird mit destilliertem Wasser gewaschen, über Magnesiumsulfat getrocknet, filtriert und eingedampft. Man erhält einen öligen Rückstand, den man in 100 ml

Äther auflöst und mit 1,8 ml einer 2N Lösung von 2-Äthylcapronsäure-Natriumsalz in Essigester versetzt. Der kristalline Niederschlag wird abgenutscht, mit Äther gewaschen und über Phosphorpentoxid im Vakuum getrocknet. Man erhält Natrium-(2S,5R)-6-acetonyliden-3,3-dimethyl-7-oxo-4-thia-1-azabicyclo[3.2.0]heptan-2-carboxylat vom Schmelzpunkt 150° (Zersetzung) [Rf: 0,31; Chloroform/Essigester/Essigsäure(5 : 4 : 1); NMR ($D_2O$), δ (ppm): 1,58 (3H); 1,62 (3H); 2,45 (3H); 4,46 (1H); 6,12 (1H); 6,8 (1H)].

Beispiel 11

Eine Lösung von 3,8 g Methylen-6,7-dioxo-3,3-dimethyl-4-thia-1-azabicyclo[3.2.0]heptan-2-carboxylat-pivalat in 100 ml Benzol wird bei 20° mit 3,5 g Formylmethylentriphenylphosphoran versetzt. Nach 10 Minuten wird das Reaktionsgemisch eingedampft. Der Rückstand wird unter Eluieren mit n-Hexan/Essigester (8 : 2) an Kieselgel chromatographiert. Man erhält Methylen-(2S,5R)-6-[(E)-formylmethylen]-3,3-dimethyl-7-oxo-4-thia-1-azabicyclo[3.2.0]heptan-2-carboxylat-pivalat [Rf: 0,27; Cyclohexan/Essigester (8 : 2); NMR ($CDCl_3$), δ (ppm): 5,68 ($1H_3$); 5,9 ($1H_5$); 6,24 (1H); 10,3 (1H)] und Methylen-(2S,5R)-6-[(Z)-formylmethylen]-3,3-dimethyl-7-oxo-4-thia-1-azabicyclo[3.2.0]heptan-2-carboxylat-pivalat [Rf: 0,2; Cyclohexan/Essigester (8 : 2); NMR ($CDCl_3$), δ (ppm): 5,68 ($1H_3$); 6,12 ($1H_5$); 6,55 (1H); 9,86 (1H)].

Beispiel 12

Eine Lösung von 1,2 g Methylen-(2S,5R)-6-acetonyliden-3,3-dimethyl-7-oxo-4-thia-1-zabicyclo[3.2.0]heptan-2-carboxylat-pivalat in 50 ml Dichlormethan wird bei 20° mit 0,6 g m-Chlorperbenzoesäure versetzt. Nach 5 Minuten wäscht man zweimal mit 5-proz. Natriumbicarbonatlösung und dreimal mit Wasser. Die organische Phase wird über Magnesiumsulfat getrocknet, filtriert und eingedampft. Der kristalline Rückstand wird aus Äther umkristallisiert. Man erhält Methylen-(2S,5R)-6-acetonyliden-3,3-dimethyl-7-oxo-4-thia-1-azabicyclo[3.2.0]heptan-2-carboxylat-pivalat-4-oxid als weisse Kristalle vom Schmelzpunkt 148–150°.

Beispiel 13

Eine Lösung von 1,5 g Methylen-6,7-dioxo-3,3-dimethyl-4-thia-1-azabicyclo[3.2.0]heptan-2-carboxylat-pivalat in 50 ml Benzol wird bei Raumtemperatur mit 3,1 g Triphenylphosphinbromcarboäthoxymethylen versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-6-[brom-(äthoxycarbonyl)methylen]-3,3-dimethyl-7-oxo-4-thia-1-azabicyclo[3.2.0]heptan-2-carboxylat-pivalat [Gemisch der (E)- und (Z)-Form] als rötliches Öl [Rf: 0,5; Cyclohexan/Essigester (6 : 4); Rf: 0,1; Cyclohexan/Essigester (9 : 1); NMR ($CDCl_3$), δ (ppm): 1,25 (9H); 1,39 (3H); 1,53 (3H); 1,61 (3H); 4,18 4,6 (2H); 4,65 und 4,68 (1H); 5,7–6,1 (3H)].

Beispiel 14

Eine Lösung von 1,0 g Methylen-6,7-dioxo-3,3-dimethyl-4-thia-1-azabicyclo[3.2.0]heptan-2-carboxylat-pivalat in 30 ml Benzol wird bei Raumtemperatur mit 1,8 g Diäthoxyphosphinylacetylmethylentriphenylphosphoran versetzt. Nach 10 Minuten wird das Reaktionsgemisch eingedampft. Der Rückstand wird unter Eluieren mit Essigester an Kieselgel chromatographiert. Man erhält Methylen-(2S,5R)-6-[3-(diäthoxyphosphinyl)-2-oxopropyliden]-3,3-dimethyl-7-oxo-4-thia-1-azabicyclo[3.2.0]heptan-2-carboxylat-pivalat als gelbes Öl [Rf: 0,35; Essigester; NMR ($CDCl_3$), δ (ppm): 1,23 (9H); 1,36 (3H); 1,51 (3H); 1,59 (3H); 3,12 und 3,42 (2H, P–$CH_2$); 4,21 (4H); 4,63 (1H); 5,90 (2H); 6,08 (1H); 6,82 (1H)].

Beispiel 15

Eine Lösung von 6,6 g Methylen-6,7-dioxo-3,3-dimethyl-4-thia-1-azabicyclo[3.2.0]heptan-2-carboxylat-pivalat in 150 ml Benzol wird bei Raumtemperatur mit 9 g Triphenylchloracetonylphosphoran versetzt. Nach 10 Minuten wird das Reaktionsgemisch eingedampft. Der Rückstand wird unter Eluieren mit Cyclohexan/Essigester (7 : 3) an Kieselgel chromatographiert. Man erhält Methylen-(2S,5R)-6-(3-chloracetonyliden)-3,3-dimethyl-7-oxo-4-thia-1-azabicyclo[3.2.0]heptan-2-carboxylat-pivalat als oranges Öl [Rf: 0,4; Cyclohexan/Essigester (6 : 4); NMR ($CDCl_3$), δ (ppm): 1,21 (9H); 1,51 (3H); 1,57 (3H); 4,25 (2H); 4,65 (1H); 5,9 (m, 2H); 6,1 (1H); 6,93 (1H)].

Beispiel 16

Eine Lösung von 6,6 g Methylen-6,7-dioxo-3,3-dimethyl-4-thia-1-azabicyclo[3.2.0]heptan-2-carboxylat-pivalat in 150 ml Benzol wird bei Raumtemperatur mit 11 g Äthoxycarbonyl(methoxyimino)acetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-6-[3-(äthoxycarbonyl)-3-(Z)-(methoxyimino)acetonyliden]-3,3-dimethyl-7-oxo-4-thia-1-azabicyclo[3.2.0]heptan-2-carboxylat-pivalat als oranges Öl [Rf: 0,51; Cyclohexan/Essigester (6 : 4); NMR ($CDCl_3$), δ (ppm): 1,23 (9H); 1,37 (m, 3H); 1,53 (3H); 1,62 (3H); 4,21 (3H, -$OCH_3$); 4,42 (m, 2H); 4,65 ($1H_3$); 5,9 (m, 2H); 6,11 (1H); 6,66 (1H)].

Beispiel 17

Eine Lösung von 1,2 g Methylen (2S,5R)-6-acetonyliden-3,3-dimethyl-7-oxo-4-thia-1-azabicyclo[3.2.0]heptan-2-carboxylat-pivalat in 50 ml Dichlormethan wird bei 20° mit 1,2 g m-Chlorperbenzoesäure versetzt. Man erhitzt während 4 Stunden unter Rückfluss zum Sieden. Das Reaktionsgemisch wird abgekühlt, mit 5-proz. Natriumbicarbonatlösung extrahiert und mit Wasser neutral gewaschen. Die organische Phase wird über Magnesiumsulfat getrocknet, filtriert und eingedampft. Man erhält Methylen-(2S,5R)-6-acetonyliden-3,3-dimethyl-7-oxo-4-thia-1-azabicyclo-[3.2.0]heptan-2-carboxylat-pivalat-4,4-dioxid als

gelbe Kristalle vom Schmelzpunkt 120–122° [Rf: 0,20; Cyclohexan/Essigester (6 : 4)].

Beispiel 18

Eine Lösung von 2,3 g Methylen-6,7-dioxo-3,3-dimethyl-4-thia-1-azabicyclo[3.2.0]heptan-2-carboxylat-pivalat in 75 ml Benzol wird bei Raumtemperatur mit 4 g Methylsulfonylacetylmethylentriphenylphosphoran versetzt. Nach 10 Minuten wird das Reaktionsgemisch eingedampft. Der Rückstand wird unter Eluieren mit Cyclohexan/Essigester (1 : 1) an Kieselgel chromatographiert. Man erhält Methylen-(2S,5R)-3,3-dimethyl-7-oxo-6-[3-methylsulfonyl-2-oxopropyliden]-4-thia-1-azabicyclo[3.2.0]heptan-2-carboxylat-pivalat als oranges Öl [Rf: 0,23; Cyclohexan/Essigester (1 : 1); NMR ($CDCl_3$); δ (ppm): 1,22 (9H); 1,5 (3H); 1,56 (3H); 3,03 (3H, $CH_3-SO_2$); 4,24 (2H, $-CH_2-SO_2$); 4,59 (1H); 6,02 (1H); 6,79 (1H)].

Beispiel 19

Eine Lösung von 5,2 g Methylen-6,7-dioxo-3,3-dimethyl-4-thia-1-azabicyclo[3.2.0]heptan-2-carboxylat-pivalat in 100 ml Benzol wird bei Raumtemperatur mit 8 g Thiomethylacetylmethylentriphenylphosphoran versetzt. Nach 10 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-3,3-dimethyl-7-oxo-6-[3-methylthio-2-oxopropyliden]-4-thia-1-azabicyclo[3.2.0]heptan-2-carboxylat-pivalat als oranges Öl [Rf: 0,53; Cyclohexan/Essigester (6 : 4); NMR ($CDCl_3$), δ (ppm): 1,21 (9H); 1,49 (3H); 1,56 (3H); 2,02 (3H, $CH_3-S-$); 2,25 (2H, $S-CH_2$); 4,53 (1H); 5,78 (2H); 5,99 (1H); 6,75 (1H)].

Beispiel 20

Man löst 34 ml Dimethylsulfoxid in 500 ml Dichlormethan, versetzt die erhaltene Lösung bei −65° und unter Rühren tropfenweise zuerst mit einer Lösung von 40 ml Trifluoressigsäureanhydrid in 300 ml Dichlormethan und dann mit einer Lösung von 79 g Methylen-(2S,5R,6S)-hydroxy-7-oxo-3,3-dimethyl-4-thia-1-azabicyclo[3.2.0]heptan-2-carboxylat-pivalat in 300 ml Dichlormethan. Die erhaltene gelbe Lösung wird anschliessend während 30 Minuten bei −65° gerührt und dann tropfenweise mit 76 ml Triäthylamin versetzt. Man lässt das Reaktionsgemisch bei Raumtemperatur stehen und versetzt die erhaltene braune Lösung mit 98 g Acetylmethylentriphenylphosphoran. Nach 10 Minuten wird das Reaktionsgemisch eingedampft. Der Rückstand wird unter Eluieren mit Cyclohexan/Essigester (7 : 3) an Kieselgel chromatographiert. Man erhält Methylen-(2S,5R)-5-acetonyliden-3,3-dimethyl-7-oxo-4-thia-1-azabicyclo[3.2.0]heptan-2-carboxylat-pivalat.

Beispiel 21

Eine Suspension von 2,2 g Methylen-(2S,5R)-3,3-dimethyl-6-(3-methyl-2-oxobutyliden)-7-oxo-4-thia-1-azabicyclo[3.2.0]heptan-2-carboxylat-pivalat in 30 ml Dimethylsulfoxid und 500 ml Phosphat-Puffer (pH 7,1) wird bei 20° mit 2 ml einer Suspension von Schweineleber-Esterase (3 mg/ml) versetzt. Man rührt während 2 Stunden bei 20° und extrahiert anschliessend das Reaktionsgemisch mit Äther. Die wässrige Phase wird mit 50-proz. Zitronensäure angesäuert (pH 3,2) und mit Ähter ausgeschüttelt. Der organische Auszug wird mit destilliertem Wasser gewaschen, über Magnesiumsulfat getrocknet, filtriert und eingedampft. Man erhält einen öligen Rückstand, den man in 200 ml Äther auflöst und mit 2,4 ml einer 2M-Lösung von 2-Äthylcapronsäure-Natriumsalz in Essigester versetzt. Der kristalline Niederschlag wird abgenutscht, mit Ähter gewaschen und über Phosphorpentoxid im Vakuum getrocknet. Man erhält Natrium-(2S,5R)-3,3-dimethyl-6-(3-methyl-2-oxobutyliden)-7-oxo-4-thia-1-azabicyclo-[3.2.0]heptan-2-carboxylat vom Schmelzpunkt 145° (Zersetzung). [$R_f$: 0,41; Chloroform/Essigester/Essigsäure(5 : 4 : 1); NMR ($D_2O$), δ (ppm); 1,17 (6H); 1,53 (3H); 1,61 (3H); 2,95 (1H); 4,4 (1H); 6,05 (1H); 6,9 (1H)].

Beispiel 22

Man löst 1,1 ml Dimethylsulfoxid in 20 ml Dichlormethan, versetzt die erhaltene Lösung bei −65° unter Rühren tropfenweise zuerst mit einer Lösung von 1,3 ml Trifluoressigsäureanhydrid in 10 ml Dichlormethan und dann mit einer Lösung von 2,4 g Methylen-(2S,5R,6S)-hydroxy-7-oxo-3,3-dimethyl-4-thia-1-azabicyclo[3.2.0]heptan-2-carboxylat-acetat in 20 ml Dichlormethan. Die erhaltene gelbe Lösung wird anschliessend während 30 Minuten bei −65° gerührt und dann tropfenweise mit 2,45 ml Triäthylamin versetzt. Man lässt das Reaktionsgemisch bei Raumtemperatur stehen und versetzt die erhaltene braune Lösung mit 3,2 g Acetylmethylentriphenylphosphoran. Nach 5 Minuten wird das Reaktionsgemisch eingedampft. Der Rückstand wird unter Eluieren mit Cyclohexan/Essigester (6 : 4) an Kieselgel chromatographiert. Man erhält Methylen-(2S,5R)-6-acetonyliden-3,3-dimethyl-7-oxo-4-thia-1-azabicyclo[3.2.0]heptan-2-carboxylat-acetat [$R_f$: 0,21; Cyclohexan/Essigester (6 : 4); NMR ($CDCl_3$), δ (ppm); 1,51 (3H); 1,59 (3H); 2,13 (3H); 2,36 (3H); 4,6 (1H); 5,83 (2H); 6,03 (1H); 6,57 (1H)].

Beispiel 23

Eine Lösung von 7,9 g Methylen-6,7-dioxo-3,3-dimethyl-4-thia-1-azabicyclo[3.2.0]heptan-2-carboxylat-pivalat in 150 ml Benzol wird bei 20° mit 10,6 g Thiophenylacetylmethylentriphenylphosphoran versetzt. Nach 5 Minuten wird das Reaktionsgemisch eingedampft. Der Rückstand wird unter Eluieren mit Cyclohexan/Essigester (7 : 3) an Kieselgel chromatographiert. Man erhält Methylen-(2S,5R)-3,3-dimethyl-7-oxo-6-[3-phenylthio-2-oxopropyliden]-4-thia-1-azabicyclo[3.2.0]heptan-2-carboxylat-pivalat als oranges Öl [$R_f$: 0,34; Cyclohexan/Essigester (7 : 3); NMR ($CDCl_3$); δ (ppm): 1,21 (9H); 1,48 (3H); 1,56 (3H); 3,73 (2H); 4,57 (1H); 5,8 (2H); 5,95 (1H); 6,88 (1H); 7,35 (5H)].

**Patentansprüche für die Vertragsstaaten BE CH DE FR GB IT LI LU NL SE**

1. β-Lactame der allgemeinen Formel

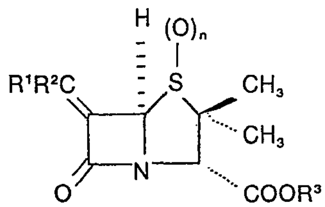

(I)

worin $R^1$ Cyano oder einen Rest der Formel $R^4$–CO– oder $R^5$–A–CO–, $R^2$ Wasserstoff, niederes Alkyl oder Halogen, $R^3$ Wasserstoff, Pivaloyloxymethyl, Acetoxymethyl, 1-Pivaloyloxyäthyl, 1-Acetoxyäthyl, Methoxycarbonyloxymethyl, 1-Äthoxycarbonyloxyäthyl, 1-Isopropoxycarbonyloxyäthyl, Phthalidyl, Thiophthalidyl, Methoxymethyl oder Acetamidomethyl, n die Zahl 0, 1 oder 2, $R^4$ Wasserstoff, Hydroxy, niederes Alkoxy, niederes Alkyl, Aryl oder einen Rest der Formel $R^6ON=C-COOR^7$, $R^5$ Halogen, niederes Alkoxy, niederes Alkylthio, niederes Alkylsulfinyl, niederes Alkylsulfonyl, Aryl, Aryloxy, Arylthio, Arylsulfinyl, Arylsulfonyl oder di-(niederes Alkoxy)-phosphinyl, $R^6$ und $R^7$ je niederes Alkyl und A niederes Alkylen bedeuten, wobei «Aryl» einen gegebenenfalls durch Halogen, Nitro, Hydroxy, niederes Alkyl oder niederes Alkoxy substituierten Phenylrest darstellt und «niedere» Gruppen höchstens 7 Kohlenstoffatome tragen,
und pharmazeutisch annehmbare Salze von Carbonsäuren der allgemeinen Formel I mit Basen.

2. Verbindungen nach Anspruch 1, dadurch gekennzeichnet, dass $R^1$ Cyano oder einen Rest der Formel $R^4$–CO– oder $R^5$–A–CO–, $R^4$ Wasserstoff, niederes Alkyl, Phenyl oder p-Nitrophenyl, $R^5$ Chlor, Methylthio, Methylsulfonyl, Phenyl, Phenoxy, Phenylthio oder Phenylsulfonyl und A Methylen bedeuten.

3. Verbindungen nach Anspruch 1 oder 2, dadurch gekennzeichnet, dass $R^2$ Wasserstoff oder Halogen bedeutet.

4. Verbindungen nach einem der Ansprüche 1 bis 3, dadurch gekennzeichnet, dass $R^3$ Wasserstoff, Acetoxymethyl oder Pivaloyloxymethyl bedeutet.

5. Verbindungen nach einem der Ansprüche 1 bis 4, dadurch gekennzeichnet, dass n die Zahl 0 bedeutet.

6. Methylen-(2S,5R)-6-[(Z)-acetonyliden]-3,3-dimethyl-7-oxo-4-thia-1-azabicyclo[3.2.0]heptan-2-carboxylat-pivalat.

7. Natrium-(2S,5R)-6-[(Z)-acetonyliden]-3,3-dimethyl-7-oxo-4-thia-1-azabicyclo[3.2.0]heptan-2-carboxylat.

8. Methylen-(2S,5R)-3,3-dimethyl-6-(3-methyl-2-oxo-butyliden)-7-oxo-4-thia-1-azabicyclo-[3.2.0]heptan-2-carboxylat-pivalat.

9. Methylen-(2S,5R)-3,3-dimethyl-7-oxo-6-(2-oxo-3-phenoxypropyliden)-4-thia-1-azabicyclo-[3.2.0]heptan-2-carboxylat-pivalat.

10. Methylen-(2S,5R)-3,3-dimethyl-7-oxo-6-(2-oxo-3-phenylpropyliden)-4-thia-1-azabicyclo-[3.2.0]heptan-2-carboxylat-pivalat.

11. Methylen-(2S,5R)-3,3-dimethyl-7-oxo-6-[3-methylthio-2-oxopropyliden]-4-thia-1-azabicyclo-[3.2.0]heptan-2-carboxylat-pivalat.

12. Methylen-(2S,5R)-6-acetonyliden-3,3-dimethyl-7-oxo-4-thia-1-azabicyclo[3.2.0]heptan-2-carboxylat-pivalat-4,4-dioxid.

13. Methylen-(2S,5R)-3,3-dimethyl-6-(1-chlor-2-oxopropyliden)-7-oxo-4-thia-1-azabicyclo-[3.2.0]heptan-2-carboxylat-pivalat.

14. Methylen-(2S,5R)-6-(3-chloracetonyliden)-3,3-dimethyl-7-oxo-4-thia-1-azabicyclo[3.2.0]heptan-2-carboxylat-pivalat.

15. Methylen-(2S,5R)-6-acetonyliden-3,3-dimethyl-7-oxo-4-thia-1-azabicyclo[3.2.0]heptan-2-carboxylat-pivalat-4-oxid.

16. (E)- und (Z)-Methylen-(2S,5R)-6-(formylmethylen)-3,3-dimethyl-7-oxo-4-thia-1-azabicyclo-[3.2.0]-heptan-2-carboxylat-pivalat.

17. Verbindungen gemäss einem der Ansprüche 1 bis 16 als pharmazeutische Wirkstoffe.

18. Verbindungen gemäss einem der Ansprüche 1 bis 16 als β-Lactamase hemmende Wirkstoffe.

19. Verfahren zur Herstellung von Verbindungen gemäss einem der Ansprüche 1 bis 16, dadurch gekennzeichnet, dass man

a) eine Verbindung der allgemeinen Formel

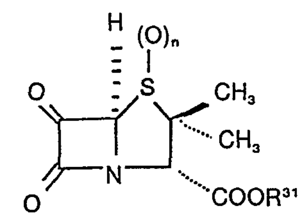

(II)

worin $R^{31}$ wie $R^3$ ist, jedoch nicht Wasserstoff darstellt, und n die in Anspruch 1 angegebene Bedeutung besitzt,
mit einem Phosphoran der allgemeinen Formel

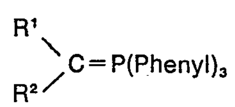

(III)

worin $R^1$ und $R^2$ die in Anspruch 1 angegebene Bedeutung besitzen,
umsetzt, oder

b) eine Verbindung der allgemeinen Formel

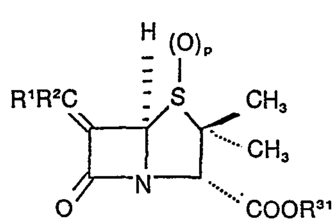

(Ia)

worin p die Zahl 0 oder 1 bedeutet, und $R^1$ und $R^2$ die in Anspruch 1 angegebene und $R^{31}$ obige Bedeutung besitzen,

am (an den) Schwefelatom(en) oxidiert, oder

c) in einer Verbindung der allgemeinen Formel

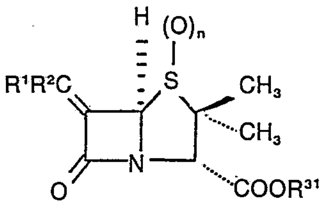

(Ib)

worin $R^1$, $R^2$ und n die in Anspruch 1 angegebene und $R^{31}$ obige Bedeutung besitzen, die Estergruppe hydrolysiert,

d) erwünschtenfalls ein erhaltenes Gemisch der (E)- und (Z)-Isomeren auftrennt, und

e) erwünschtenfalls eine erhaltene Carbonsäure der allgemeinen Formel I mit einer Base in ein pharmazeutisch annehmbares Salz überführt.

20. Arzneimittel, enthaltend eine Verbindung gemäss einem der Ansprüche 1 bis 16.

21. β-Lactamase hemmende Arzneimittel, enthaltend eine Verbindung gemäss einem der Ansprüche 1 bis 16.

22. Arzneimittel gemäss Anspruch 20 oder 21, enthaltend ein β-Lactam-Antibiotikum.

23. Arzneimittel gemäss Anspruch 22, enthaltend ein Penicillin oder Cephalosporin als β-Lactam-Antibiotikum.

24. Arzneimittel gemäss Anspruch 23, enthaltend Benzylpenicillin, Phenoxymethylpenicillin, Carbenicillin, Methicillin, Propicillin, Ampicillin, Amoxicillin, Mecillinam, Cephaloridin, Cephalotin, Cefazolin, Cephalexin, Cefoxitin, Cephacetril, Cephamandol, Cephapirin, Cephradin oder Cephaloglycin.

25. Verbindungen der allgemeinen Formel

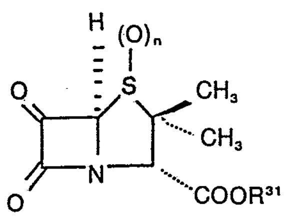

(II)

worin $R^{31}$ wie $R^3$ ist, jedoch nicht Wasserstoff darstellt, und n die Zahl 0, 1 oder 2 bedeuten.

**Patentansprüche für den Vertragsstaat AT**

1. Verfahren zur Herstellung von β-Lactamen der allgemeinen Formel

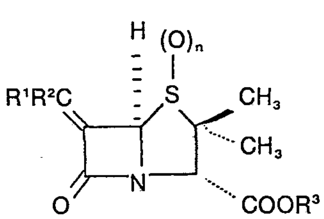

(I)

worin $R^1$ Cyano oder einen Rest der Formel $R^4$–CO– oder $R^5$–A–CO–, $R^2$ Wasserstoff, niederes Alkyl oder Halogen, $R^3$ Wasserstoff, Pivaloyloxymethyl, Acetoxymethyl, 1-Pivaloyloxyäthyl, 1-Acetoxyäthyl, Methoxycarbonyloxymethyl, 1-Äthoxycarbonyloxyäthyl, 1-Isopropoxycarbonyloxyäthyl, Phthalidyl, Thiophthalidyl, Methoxymethyl oder Acetamidomethyl, n die Zahl 0, 1 oder 2, $R^4$ Wasserstoff, Hydroxy, niederes Alkoxy, niederes Alkyl, Aryl oder einen Rest der Formel $R^6ON{=}C{-}COOR^7$, $R^5$ Halogen, niederes Alkoxy, niederes Alkylthio, niederes Alkylsulfinyl, niederes Alkylsulfonyl, Aryl, Aryloxy, Arylthio, Arylsulfinyl, Arylsulfonyl oder di-(niederes Alkoxy)-phosphinyl, $R^6$ und $R^7$ je niederes Alkyl und A niederes Alkylen bedeuten, wobei «Aryl» einen gegebenenfalls durch Halogen, Nitro, Hydroxy, niederes Alkyl oder niederes Alkoxy substituierten Phenylrest darstellt und «niedere» Gruppen höchstens 7 Kohlenstoffatome tragen,

und pharmazeutisch annehmbaren Salze von Carbonsäuren der allgemeinen Formel I mit Basen, dadurch gekennzeichnet, dass man

a) eine Verbindung der allgemeinen Formel

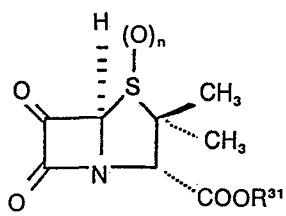

(II)

worin $R^{31}$ wie $R^3$ ist, jedoch nicht Wasserstoff darstellt, und n obige Bedeutung besitzt, mit einem Phosphoran der allgemeinen Formel

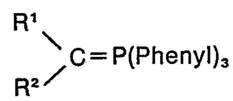

(III)

worin $R^1$ und $R^2$ obige Bedeutung besitzen, umsetzt, oder

b) eine Verbindung der allgemeinen Formel

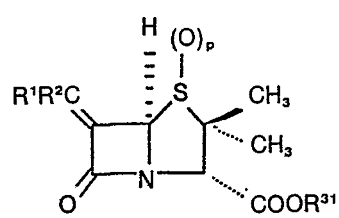

(Ia)

worin p die Zahl 0 oder 1 bedeutet, und $R^1$, $R^2$ und $R^{31}$ obige Bedeutung besitzen, am (an den) Schwefelatom(en) oxidiert, oder

c) in einer Verbindung der allgemeinen Formel

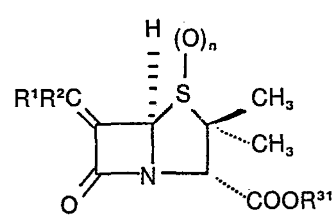

(Ib)

worin $R^1$ $R^2$, n und $R^{31}$ obige Bedeutung besitzen, die Estergruppe hydrolysiert,

d) erwünschtenfalls ein erhaltenes Gemisch der (E)- und (Z)-Isomeren auftrennt, und

e) erwünschtenfalls eine erhaltene Carbonsäure der allgemeinen Formel I mit einer Base in ein pharmazeutisch annehmbares Salz überführt.

2. Verfahren nach Anspruch 1, dadurch gekennzeichnet, dass man eine Verbindung der in Anspruch 1 definierten Formel I herstellt, worin $R^1$ Cyano oder einen Rest der Formel $R^4$–CO– oder $R^5$–A–CO–, $R^4$ Wasserstoff, niederes Alkyl, Phenyl oder p-Nitrophenyl, $R^5$ Chlor, Methylthio, Methylsulfonyl, Phenyl, Phenoxy, Phenylthio oder Phenylsulfonyl und A Methylen bedeuten.

3. Verfahren nach Anspruch 1 oder 2, dadurch gekennzeichnet, dass man eine Verbindung der in Anspruch 1 definierten Formel I herstellt, worin $R^2$ Wasserstoff oder Halogen bedeutet.

4. Verfahren nach einem der Ansprüche 1 bis 3, dadurch gekennzeichnet, dass man eine Verbindung der in Anspruch 1 definierten Formel I herstellt, worin $R^3$ Wasserstoff, Acetoxymethyl oder Pivaloyloxymethyl bedeutet.

5. Verfahren nach einem der Ansprüche 1 bis 4, dadurch gekennzeichnet, dass man eine Verbindung der in Anspruch 1 definierten Formel I herstellt, worin n die Zahl 0 bedeutet.

6. Verfahren nach Anspruch 1, dadurch gekennzeichnet, dass man Methylen-(2S,5R)-6-[(Z)-acetonyliden]-3,3-dimethyl-7-oxo-4-thia-1-azabicyclo[3.2.0]heptan-2-carboxylatpivalat herstellt.

7. Verfahren nach Anspruch 1, dadurch gekennzeichnet, dass man Natrium-(2S,5R)-6-[(Z)-acetonyliden]-3,3-dimethyl-7-oxo-4-thia-1-azabicyclo-[3.2.0]heptan-2-carboxylat herstellt.

8. Verfahren nach Anspruch 1, dadurch gekennzeichnet, dass man Methylen-(2S,5R)-3,3-dimethyl-6-(3-methyl-2-oxo-butyliden)-7-oxo-4-thia-1-azabicyclo[3.2.0]heptan-2-carboxylat-pivalat herstellt.

9. Verfahren nach Anspruch 1, dadurch gekennzeichnet, dass man Methylen-(2S,5R)-3,3-dimethyl-7-oxo-6-(2-oxo-3-phenoxypropyliden)-4-thia-1-azabicyclo[3.2.0]heptan-2-carboxylat-pivalat herstellt.

10. Verfahren nach Anspruch 1, dadurch gekennzeichnet, dass man Methylen-(2S,5R)-3,3-dimethyl-7-oxo-6-(2-oxo-3-phenylpropyliden)-4-thia-1-azabicyclo[3.2.0]heptan-2-carboxylat-pivalat herstellt.

11. Verfahren nach Anspruch 1, dadurch gekennzeichnet, dass man Methylen-(2S,5R)-3,3-dimethyl-7-oxo-6-[3-methyl-thio-2-oxopropyliden]-4-thia-1-azabicyclo[3.2.0]heptan-2-carboxylat-pivalat herstellt.

12. Verfahren nach Anspruch 1, dadurch gekennzeichnet, dass man Methylen-(2S,5R)-6-acetonyliden-3,3-dimethyl-7-oxo-4-thia-1-azabicyclo[3.2.0]heptan-2-carboxylat-pivalat-4,4-dioxid herstellt.

13. Verfahren nach Anspruch 1, dadurch gekennzeichnet, dass man Methylen-(2S,5R)-3,3-dimethyl-6-(1-chlor-2-oxopropyliden)-7-oxo-4-thia-1-azabicyclo[3.2.0]heptan-2-carboxylat-pivalat herstellt.

14. Verfahren nach Anspruch 1, dadurch gekennzeichnet, dass man Methylen-(2S,5R)-6-(3-chloracetonyliden)-3,3-dimethyl-7-oxo-4-thia-1-azabicyclo[3.2.0]heptan-2-carboxylat-pivalat herstellt.

15. Verfahren nach Anspruch 1, dadurch gekennzeichnet, dass man Methylen-(2S,5R)-6-acetonyliden-3,3-dimethyl-7-oxo-4-thia-1-azabicyclo[3.2.0]heptan-2-carboxylat-pivalat-4-oxid herstellt.

16. Verfahren nach Anspruch 1, dadurch gekennzeichnet, dass man (E)- und (Z)-Methylen-(2S,5R)-6-(formyl-methylen)-3,3-dimethyl-7-oxo-4-thia-1-azabicyclo[3.2.0]heptan-2-carboxylat-pivalat herstellt.

17. Verfahren zur Herstellung von Verbindungen der allgemeinen Formel

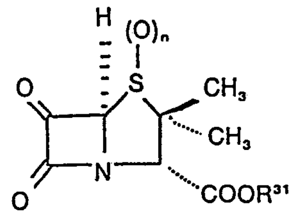

(II)

worin $R^{31}$ wie $R^3$ ist, jedoch nicht Wasserstoff darstellt, und n die Zahl 0, 1 oder 2 bedeuten, dadurch gekennzeichnet, dass man eine Verbindung der allgemeinen Formel

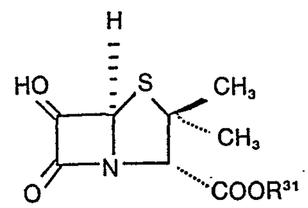

(V)

worin $R^{31}$ obige Bedeutung besitzt oxidiert.

18. Verfahren nach Anspruch 17 zur Herstellung einer Verbindung der Formel II worin n die Zahl 0 darstellt, dadurch gekennzeichnet, dass man die Verbindung der Formel V mit Trifluoressigsäureanhydrid und Dimethylsulfoxid oxidiert.

19. Verfahren nach Anspruch 18, dadurch gekennzeichnet, dass man das erhaltene Produkt mit einem Peroxid in eine Verbindung der Formel II, worin n die Zahl 1 oder 2 darstellt, überführt.

20. Verfahren nach Anspruch 19, dadurch gekennzeichnet, dass man als Peroxid m-Chlorperbenzoesäure verwendet.

**Claims for the Contracting States BE CH DE FR GB IT LI LU NL SE**

1. β-Lactams of the general formula

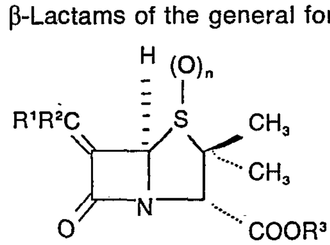

(I)

wherein $R^1$ signifies cyano or a residue of the formula $R^4$–CO– or $R^5$–A–CO–, $R^2$ signifies hydrogen, lower alkyl or halogen, $R^3$ signifies hydrogen, pivaloyloxymethyl, acetoxymethyl, 1-pivaloyloxyethyl, 1-acetoxyethyl, methoxycarbonyloxymethyl, 1-ethoxycarbonyloxyethyl, 1-isopropoxycarbonyloxyethyl, phthalidyl, thiophthalidyl, methoxymethyl or acetamidomethyl, n signifies the number 0, 1 or 2, $R^4$ signifies hydrogen, hydroxy, lower alkoxy, lower alkyl, aryl or a residue of the formula $R^6ON=C–COOR^7$, $R^5$ signifies halogen, lower alkoxy, lower alkylthio, lower alkylsulphinyl, lower alkylsulphonyl, aryl, aryloxy, arylthio, arylsulphinyl, arylsulphonyl or di-(lower alkoxy)-phosphinyl, $R^6$ and $R^7$ each signify lower alkyl and A signifies lower alkylene, whereby «aryl» represents a phenyl residue optionally substituted by halogen, nitro, hydroxy, lower alkyl or lower alkoxy and «lower» groups have at most 7 carbon atoms,
and pharmaceutically acceptable salts of carboxylic acids of general formula I with bases.

2. Compounds according to claim 1, characterized in that $R^1$ signifies cyano or a residue of the formula $R^4$–CO– or $R^5$–A–CO–, $R^4$ signifies hydrogen, lower alkyl, phenyl or p-nitrophenyl, $R^5$ signifies chlorine, methylthio, methylsulphonyl, phenyl, phenoxy, phenylthio or phenylsulphonyl and A signifies methylene.

3. Compounds according to claim 1 or 2, characterized in that $R^2$ signifies hydrogen or halogen.

4. Compounds according to any one of claims 1 to 3, characterized in that $R^3$ signifies hydrogen, acetoxymethyl or pivaloyloxymethyl.

5. Compounds according to any one of claims 1 to 4, characterized in that n signifies the number 0.

6. Methylene (2S,5R)-6-[(Z)-acetonylidene]-3,3-dimethyl-7-oxo-4-thia-1-azabicyclo[3.2.0]heptane-2-carboxylate pivalate.

7. Sodium (2S,5R)-6-[(Z)-acetonylidene]-3,3-dimethyl-7-oxo-4-thia-1-azabicyclo[3.2.0]heptane-2-carboxylate.

8. Methylene (2S,5R)-3,3-dimethyl-6-(3-methyl-2-oxo-butylidene)-7-oxo-4-thia-1-azabicyclo-[3.2.0]heptane-2-carboxylate pivalate.

9. Methylene (2S,5R)-3,3-dimethyl-7-oxo-6-(2-oxo-3-phenoxypropylidene)-4-thia-1-azabicyclo-[3.2.0]heptane-2-carboxylate pivalate.

10. Methylene (2S,5R)-3,3-dimethyl-7-oxo-6-(2-oxo-3-phenylpropylidene)-4-thia-1-azabicyclo-[3.2.0]heptane-2-carboxylate pivalate.

11. Methylene (2S,5R)-3,3-dimethyl-7-oxo-6-[3-methyl-thio-2-oxopropylidene]-4-thia-1-azabicyclo[3.2.0]heptane-2-carboxylate pivalate.

12. Methylene (2S,5R)-6-acetonylidene-3,3-dimethyl-7-oxo-4-thia-1-azabicyclo[3.2.0]heptane-2-carboxylate pivalate 4,4-dioxide.

13. Methylene (2S,5R)-3,3-dimethyl-6-(1-chloro-2-oxo-propylidene)-7-oxo-4-thia-1-azabicyclo-[3.2.0]heptane-2-carboxylate pivalate.

14. Methylene (2S,5R)-6-(3-chloroacetonylidene)-3,3-dimethyl-7-oxo-4-thia-1-azabicyclo-[3.2.0]heptane-2-carboxylate pivalate.

15. Methylene (2S,5R)-6-acetonylidene-3,3-dimethyl-7-oxo-4-thia-1-azabicyclo[3.2.0]heptane-2-carboxylate pivalate 4-oxide.

16. (E)- and (Z)-Methylene (2S,5R)-6-(formylmethylene)-3,3-dimethyl-7-oxo-4-thia-1-azabicyclo-[3.2.0]heptane-2-carboxylate pivalate.

17. Compounds in accordance with any one of claims 1 to 16 as pharmaceutically active substances.

18. Compounds in accordance with any one of claims 1 to 16 as β-lactamase-inhibiting active substances.

19. A process for the manufacture of compounds in accordance with any one of claims 1 to 16, characterized by

a) reacting a compound of the general formula

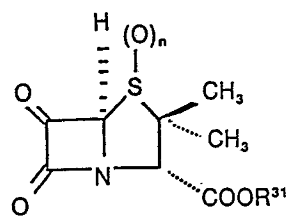

(II)

wherein $R^{31}$ is the same as $R^3$, but does not represent hydrogen, and n has the significance given in claim 1,
with a phosphorane of the general formula

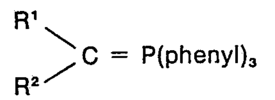

(III)

wherein $R^1$ and $R^2$ have the significance given in claim 1,
or

b) oxidizing a compound of the general formula

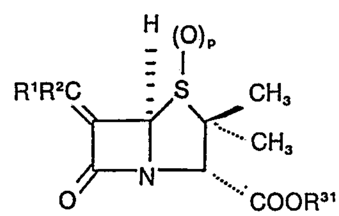

(Ia)

wherein p signifies the number 0 or 1, and $R^1$ and $R^2$ have the significance given in claim 1 and $R^{31}$ has the above significance,
at the sulphur atom(s), or

c) hydrolyzing the ester group in a compound of the general formula

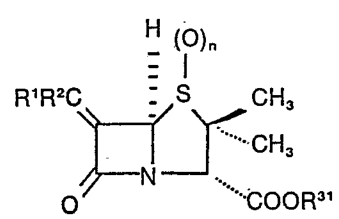

(Ib)

wherein $R^1$, $R^2$ and n have the significance given in claim 1 and $R^{31}$ has the above significance,

d) if desired, separating a mixture of the (E) and (Z) isomers obtained, and

e) if desired, converting a carboxylic acid of general formula I obtained with a base into a pharmaceutically acceptable salt.

20. A medicament containing a compound in accordance with any one of claims 1 to 16.

21. A β-lactamase-inhibiting medicament containing a compound in accordance with any one of claims 1 to 16.

22. A medicament in accordance with claim 20 or 21, containing a β-lactam antibiotic.

23. A medicament in accordance with claim 22 containing a penicillin or cephalosporin as the β-lactam antibiotic.

24. A medicament in accordance with claim 23 containing benzylpenicillin, phenoxymethylpenicillin, carbenicillin, methicillin, propicillin, ampicillin, amoxicillin, mecillinam, cephaloridine, cephalothin, cefazolin, cephalexin, cefoxitin, cephacetrile, cephamandole, cephapirin, cephradine or cephaloglycin.

25. Compounds of the general formula

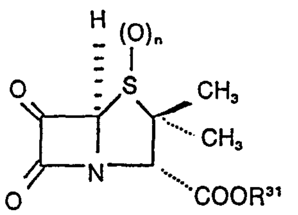

(II)

wherein $R^{31}$ is the same as $R^3$, but does not represent hydrogen, and n signifies the number 0, 1 or 2.

**Claims for the Contracting State AT**

1. A process for the manufacture of β-lactams of the general formula

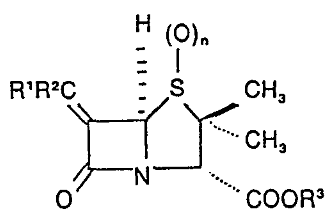

(I)

wherein $R^1$ signifies cyano or a residue of the formula $R^4$–CO– or $R^5$–A–CO–, $R^2$ signfies hydrogen, lower alkyl or halogen, $R^3$ signifies hydrogen, pivaloyloxymethyl, acetoxymethyl, 1-pivaloyloxyethyl, 1-acetoxyethyl, methoxycarbonyloxymethyl, 1-ethoxycarbonyloxyethyl, 1-isopropoxycarbonyloxyethyl, phthalidyl, thiophthalidyl, methoxymethyl or acetamidomethyl, n signifies the number 0, 1 or 2, $R^4$ signifies hydrogen, hydroxy, lower alkoxy, lower alkyl, aryl or a residue of the formula $R^6ON{=}C{-}COOR^7$, $R^5$ signifies halogen, lower alkoxy, lower alkylthio, lower alkylsulphinyl, lower alkylsulphonyl, aryl, aryloxy, arylthio, arylsulphinyl, arylsulphonyl or di-(lower alkoxy)-phosphinyl, $R^6$ and $R^7$ each signify lower alkyl and A signifies lower alkylene, whereby «aryl» represents a phenyl residue optionally substituted by halogen, nitro, hydroxy, lower alkyl or lower alkoxy and «lower» groups have at most 7 carbon atoms,

and pharmaceutically acceptable salts of carboxylic acids of general formula I with bases, characterized by

a) reacting a compound of the general formula

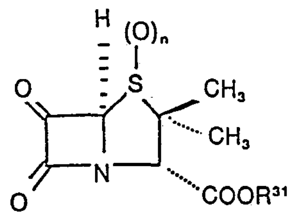

(II)

wherein $R^{31}$ is the same as $R^3$, but does not represent hydrogen, and n has the above significance,

with a phosporane of the general formula

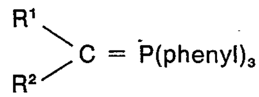

(III)

wherein $R^1$ and $R^2$ have the above significance, or

b) oxidizing a compound of the general formula

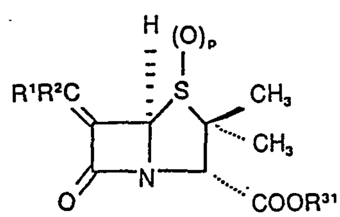

(Ia)

wherein p signifies the number 0 or 1 and $R^1$, $R^2$ and $R^{31}$ have the above significance, at the sulphur atom(s), or

c) hydrolyzing the ester group in a compound of the general formula

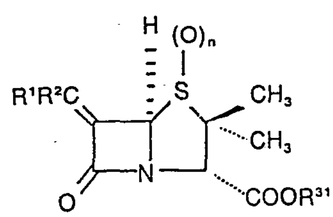

(Ib)

wherein $R^1$, $R^2$, n and $R^{31}$ have the above significance,

d) if desired, separating a mixture of the (E) and (Z) isomers obtained, and

e) if desired, converting a carboxylic acid of general formula I obtained with a base into a pharmaceutically acceptable salt.

2. A process according to claim 1, characterized in that a compound of formula I defined in claim 1 in which $R^1$ signifies cyano or a residue of the formula $R^4$–CO– or $R^5$–A–CO–, $R^4$ signifies hydrogen, lower alkyl, phenyl or p-nitrophenyl, $R^5$

signifies chlorine, methylthio, methylsulphonyl, phenyl, phenoxy, phenylthio or phenylsulphonyl and A signifies methylene is manufactured.

3. A process according to claim 1 or 2, characterized in that a compound of formula I defined in claim 1 in which $R^2$ signifies hydrogen or halogen is manufactured.

4. A process according to any one of claims 1 to 3, wherein a compound of formula I defined in claim 1 in which $R^3$ signifies hydrogen, acetoxymethyl or pivaloyloxymethyl is manufactured.

5. A process according to any one of claims 1 to 4, characterized in that a compound of formula I defined in claim 1 in which n signifies the number 0 is manufactured.

6. A process according to claim 1, characterized in that methylene (2S,5R)-6-[(Z)-acetonylidene]-3,3-dimethyl-7-oxo-4-thia-1-azabicyclo[3.2.0]heptane-2-carboxylate pivalate is manufactured.

7. A process according to claim 1, characterized in that sodium (2S,5R)-6-[(Z)-acetonylidene]-3,3-dimethyl-7-oxo-4-thia-1-azabicyclo[3.2.0]heptane-2-carboxylate is manufactured.

8. A process according to claim 1, characterized in that methylene (2S,5R)-3,3-dimethyl-6-(3-methyl-2-oxobutylidene)-7-oxo-4-thia-1-azabicyclo-[3.2.0]heptane-2-carboxylate pivalate is manufactured.

9. A process according to claim 1, characterized in that methylene (2S,5R)-3,3-dimethyl-7-oxo-6-(2-oxo-3-phenoxypropylidene)-4-thia-1-azabicyclo-[3.2.0]heptane-2-carboxylate pivalate is manufactured.

10. A process according to claim 1, characterized in that methylene (2S,5R)-3,3-dimethyl-7-oxo-6-(2-oxo-3-phenylpropylidene)-4-thia-1-azabicyclo[3.2.0]heptane-2-carboxylate pivalate is manufactured.

11. A process according to claim 1, characterized in that methylene (2S,5R)-3,3-dimethyl-7-oxo-6-[3-methylthio-2-oxopropylidene]-4-thia-1-azabicyclo[3.2.0]heptane-2-carboxylate pivalate is manufactured.

12. A process according to claim 1, characterized in that methylene (2S,5R)-6-acetonylidene-3,3-dimethyl-7-oxo-4-thia-1-azabicyclo-[3.2.0]heptane-2-carboxylate pivalate 4,4-dioxide is manufactured.

13. A process according to claim 1, characterized in that methylene (2S,5R)-3,3-dimethyl-6-(1-chloro-2-oxopropylidene)-7-oxo-4-thia-1-azabicyclo[3.2.0]heptane-2-carboxylate pivalate is manufactured.

14. A process according to claim 1, characterized in that methylene (2S,5R)-6-(3-chloroacetonylidene)-3,3-dimethyl-7-oxo-4-thia-1-azabicyclo[3.2.0.]heptane-2-carboxylate pivalate is manufactured.

15. A process according to claim 1, characterized in that methylene (2S,5R)-6-acetonylidene-3,3-dimethyl-7-oxo-4-thia-1-azabicyclo [3.2.0]heptane-2-carboxylate pivalate 4-oxide is manufactured.

16. A process according to claim 1, characterized in that (E)- and (Z)-methylene (2S,5R)-6-(formylmethylene)-3,3-dimethyl-7-oxo-4-thia-1-azabicyclo[3.2.0]heptane-2-carboxylate pivalate is manufactured.

17. A process for the manufacture of compounds of the general formula

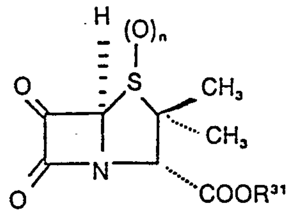
(II)

wherein $R^{31}$ is the same as $R^3$, but does not represent hydrogen, and n signifies the number 0, 1 or 2,
characterized by oxidizing a compound of the general formula

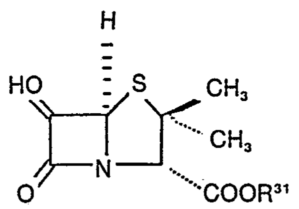
(V)

wherein $R^{31}$ has the above significance.

18. A process according to claim 17 for the manufacture of a compound of formula II in which n represents the number 0, characterized by oxidizing the compound of formula V with trifluoroacetic acid anhydride and dimethyl sulphoxide.

19. A process according to claim 18, characterized in that the product obtained is converted with a peroxide into a compound of formula II in which n represents the number 1 or 2.

20. A process according to claim 19, characterized in that m-chloroperbenzoic acid is used as the peroxide.

**Revendications pour les Etats contractants BE, CH, DE, FR, GB, IT, LI, LU, NL, SE**

1. Bêta-lactames de formule générale

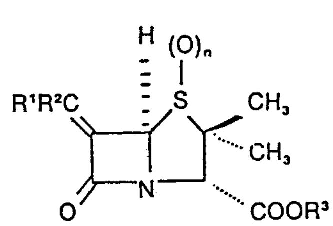
(I)

dans laquelle $R^1$ représente un groupe cyano ou un reste de formule $R^4$–CO– ou $R^5$–A–CO–, $R^2$ représente l'hydrogène, un groupe alkyle inférieur ou un halogène, $R^3$ représente l'hydrogène, un groupe pivaloyloxyméthyle, acétoxyméthyle, 1-pivaloyloxyéthyle, 1-acétoxyéthyle, méthoxycarbonyloxyméthyle, 1-éthoxycarbonyloxyéthyle, 1-isopropoxycarbonyloxyéthyle, phtalidyle, thiophta-

lidyle, méthoxyméthyle ou acétamidométhyle, n est égal à 0, 1 ou 2, $R^4$ représente l'hydrogène, un groupe hydroxy, alcoxy inférieur, alkyle inférieur, aryle ou un reste de formule $R^6ON=C-COOR^7$, $R^5$ représente un halogène, un groupe alcoxy inférieur, alkylthio inférieur, alkylsulfinyle inférieur, alkylsulfonyle inférieur, aryle, aryloxy, arylthio, arylsulfinyle, arylsulfonyle ou di-(alcoxy inférieur)-phosphinyle, $R^6$ et $R^7$ représentent chacun un groupe alkyle inférieur et A un groupe alkylène inférieur, l'expression «aryle» désignant un groupe phényle éventuellement substitué par des halogènes, des groupes nitro, hydroxy, alkyle inférieurs ou alcoxy inférieurs, et les groupes «inférieurs» contenant au maximum 7 atomes de carbone,
et les sels acceptables pour l'usage pharmaceutique des acides carboxyliques de formule générale I et de bases.

2. Composés selon la rev. 1, caractérisés en ce que $R^1$ représente un groupe cyano ou un reste de formule $R^4-CO-$ ou $R^5-A-CO-$, $R^4$ représente l'hydrogène, un groupe alkyle inférieur, phényle ou p-nitrophényle, $R^5$ représente le chlore, un groupe méthylthio, méthylsulfonyle, phényle, phénoxy, phénylthio ou phénylsulfonyle et A représente le groupe méthylène.

3. Composés selon la rev. 1 ou 2, caractérisés en ce que $R^2$ représente l'hydrogène ou un halogène.

4. Composés selon l'une des rev. 1 à 3, caractérisés en ce que $R^3$ représente l'hydrogène, un groupe acétoxyméthyle ou pivaloyloxyméthyle.

5. Composés selon l'une des rev. 1 à 4, caractérisés en ce que n est égal à 0.

6. Le méthylène-(2S,5R)-6-[(Z)-acétonylidène]-3,3-diméthyl-7-oxo-4-thia-1-azabicyclo[3.2.0]heptane-2-carboxylate-pivalate.

7. Le (2S,5R)-6-[(Z)-acétonylidène]-3,3-diméthyl-7-oxo-4-thia-1-azabicyclo[3.2.0]heptane-2-carboxylate de sodium.

8. Le méthylène-(2S,5R)-3,3-diméthyl-6-(3-méthyl-2-oxobutylidène)-7-oxo-4-thia-1-azabicyclo[3.2.0]heptane-2-carboxylate-pivalate.

9. Le méthylène-(2S,5R)-3,3-diméthyl-7-oxo-6-(2-oxo-3-phénoxypropylidène)-4-thia-1-azabicyclo[3.2.0]heptane-2-carboxylate-pivalate.

10. Le méthylène-(2S,5R)-3,3-diméthyl-7-oxo-6-(2-oxo-3-phénylpropylidène)-4-thia-1-azabicyclo-[3.2.0]heptane-2-carboxylate-pivalate.

11. Le méthylène-(2S,5R)-3,3-diméthyl-7-oxo-6-(3-méthylthio-2-oxopropylidène)-4-thia-1-azabicyclo[3.2.0]heptane-2-carboxylate-pivalate.

12. Le 4,4-dioxyde du méthylène-(2S,5R)-6-acétonylidène-3,3-diméthyl-7-oxo-4-thia-1-azabicyclo[3.2.0]heptane-2-carboxylate-pivalate.

13. Le méthylène-(2S,5R)-3,3-diméthyl-6-(1-chloro-2-oxopropylidène)-7-oxo-4-thia-1-azabicyclo[3.2.0]heptane-2-carboxylate-pivalate.

14. Le méthylène-(2S,5R)-6-(3-chloracétonylidène)-3,3-diméthyl-7-oxo-4-thia-1-azabicyclo-[3.2.0]heptane-2-carboxylate-pivalate.

15. Le 4-oxyde du méthylène-(2S,5R)-6-acétonylidène-3,3-diméthyl-7-oxo-4-thia-1-azabicyclo-[3.2.0]heptane-2-carboxylate-pivalate.

16. Le (E)- et le (Z)-méthylène-(2S,5R)-6-(formylméthylène)-3,3-diméthyl-7-oxo-4-thia-1-azabicyclo[3.2.0]heptane-2-carboxylate-pivalate.

17. Composés selon l'une des rev. 1 à 16, en tant que substances actives pharmaceutiques.

18. Composés selon l'une des rev. 1 à 16, en tant que substances actives inhibant les bêta-lactamases.

19. Procédé de préparation des composés selon l'une des rev. 1 à 16, caractérisé en ce que:

a) on fait réagir un composé de formule générale

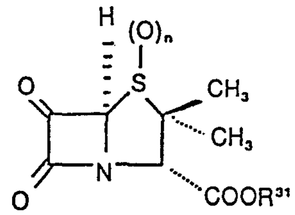

(II)

dans laquelle $R^{31}$ a les mêmes significations que $R^3$ à l'exception de l'hydrogène, et n a les significations indiquées dans la rev. 1,
avec un phosphorane de formule générale

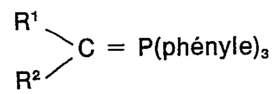

(III)

dans laquelle $R^1$ et $R^2$ ont les significations indiquées dans la rev. 1, ou bien

b) on oxyde un composé de formule générale

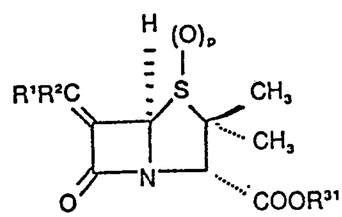

(Ia)

dans laquelle p est égal à 0 ou 1 et $R^1$ et $R^2$ ont les significations indiquées dans la rev. 1, $R^{31}$ ayant la signification indiquée ci-dessus,
sur le ou les atomes de soufre, ou bien

c) dans un composé de formule générale

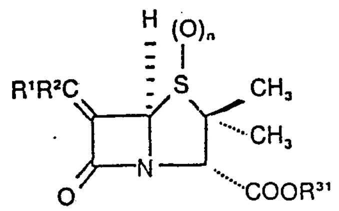

(Ib)

dans laquelle $R^1$, $R^2$ et n ont les significations indiquées dans la rev. 1, et $R^{31}$ a les significations indiquées ci-dessus,
on hydrolyse le groupe ester,

d) si on le désire, on sépare un mélange des isomères E et Z ainsi obtenu, et

e) si on le désire, on convertit un acide carboxylique de formule générale I ainsi obtenu en un sel acceptable pour l'usage pharmaceutique à l'aide d'une base.

20. Médicament contenant un composé selon l'une des rev. 1 à 16.

21. Médicament inhibiteur des bêta-lactamases, contenant un composé selon l'une des rev. 1 à 16.

22. Médicament selon la rev. 20 ou 21, contenant un antibiotique du type bêta-lactame.

23. Médicament selon la rev. 22, contenant une pénicilline ou une céphalosporine en tant qu'antibiotique du type bêta-lactame.

24. Médicament selon la rev. 23, contenant de la benzylpénicilline, de la phénoxyméthylpénicilline, de la carbénicilline, de la méthicilline, de la propicilline, de l'ampicilline, de l'amoxicilline, du mécillinam, de la céphaloridine, de la céphalotine, de la céfazoline, de la céphalexine, de la céfoxitine, du céphacétril, du céphamandol, de la céphapirine, de la céphradine ou de la céphaloglycine.

25. Composés de formule générale

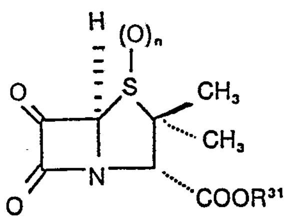

(II)

dans laquelle $R^{31}$ a les mêmes significations que $R^3$ à l'exception de l'hydrogène, et n est égal à 0, 1 ou 2.

**Revendications pour l'Etat contractant AT**

1. Procédé de préparation de bêta-lactames de formule générale

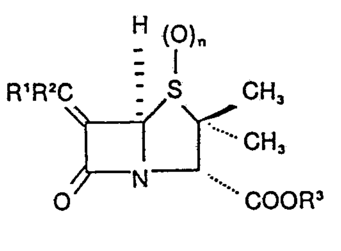

(I)

dans laquelle $R^1$ représente un groupe cyano ou un reste de formule $R^4$–CO– ou $R^5$–A–CO–, $R^2$ représente l'hydrogène, un groupe alkyle inférieur ou un halogène, $R^3$ représente l'hydrogène, un groupe pivaloyloxyméthyle, acétoxyméthyle, 1-pivaloyloxyéthyle, 1-acétoxyéthyle, méthoxycarbonyloxyméthyle, 1-éthoxycarbonyloxyéthyle, 1-isopropoxycarbonyloxyéthyle, phtalidyle, thiophtalidyle, méthoxyméthyle ou acétamidométhyle, n est égal à 0, 1 ou 2, $R^4$ représente l'hydrogène, un groupe hydroxy, alcoxy inférieur, alkyle inférieur, aryle ou un reste de formule $R^6ON=C(-)-COOR^7$, $R^5$ représente un halogène, un groupe alcoxy inférieur, alkylthio inférieur, alkylsulfinyle inférieur, alkylsulfonyle inférieur, aryle, aryloxy, arylthio, arylsulfinyle, arylsulfonyle ou di-(alcoxy inférieur)-phosphinyle, $R^6$ et $R^7$ représentent chacun un groupe alkyle inférieur et A un groupe alkylène inférieur, l'expression «aryle» désignant un groupe phényle éventuellement substitué par des halogènes, des groupes nitro, hydroxy, alkyle inférieurs ou alcoxy inférieurs, et les groupes «inférieurs» contenant au maximum 7 atomes de carbone,

et des sels acceptables pour l'usage pharmaceutique des acides carboxyliques de formule générale I et de bases, caractérisé en ce que:

a) on fait réagir un composé de formule générale

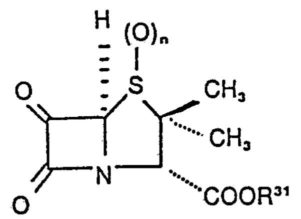

(II)

dans laquelle $R^{31}$ a les mêmes significations que $R^3$ à l'exception de l'hydrogène, et n a la signification indiquée ci-dessus,
avec un phosphorane de formule générale

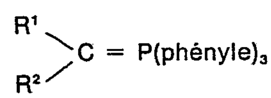

(III)

dans laquelle $R^1$ et $R^2$ ont les significations indiquées ci-dessus, ou bien

b) on oxyde un composé de formule générale

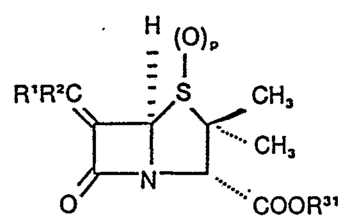

(Ia)

dans laquelle p est égal à 0 ou 1, et $R^1$, $R^2$ et $R^{31}$ ont les significations indiquées ci-dessus, sur le ou les atomes de soufre, ou bien

c) dans un composé de formule générale

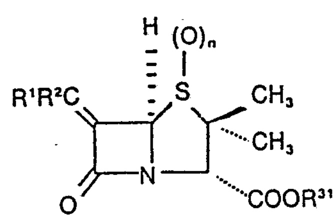

(Ib)

dans laquelle $R^1$, $R^2$, n et $R^{31}$ ont les significations indiquées ci-dessus, on hydrolyse le groupe ester,

d) si on le désire, on sépare un mélange des isomères E et Z ainsi obtenu, et

e) si on le désire, on convertit un acide carboxylique de formule générale I ainsi obtenu en un sel acceptable pour l'usage pharmaceutique à l'aide d'une base.

2. Procédé selon la rev. 1, caractérisé en ce que l'on prépare un composé de formule I définie dans la rev. 1, dans laquelle $R^1$ représente un groupe cyano ou un reste de formule $R^4$–CO– ou $R^5$–A–CO–, $R^4$ représente l'hydrogène, un groupe alkyle inférieur, phényle ou p-nitrophényle, $R^5$ représente le chlore, un groupe méthylthio, méthylsulfonyle, phényle, phénoxy, phénylthio ou phénylsulfonyle et A représente un groupe méthylène.

3. Procédé selon la rev. 1 ou 2, caractérisé en ce que l'on prépare un composé de formule I définie dans la rev. 1, dans laquelle $R^2$ représente l'hydrogène ou un halogène.

4. Procédé selon l'une des rev. 1 à 3, caractérisé en ce que l'on prépare un composé de formule I définie dans la rev. 1, dans laquelle $R^3$ représente l'hydrogène, un groupe acétoxyméthyle ou pivaloyloxyméthyle.

5. Procédé selon l'une des rev. 1 à 4, caractérisé en ce que l'on prépare un composé de formule I définie dans la rev. 1, dans laquelle n est égal à 0.

6. Procédé selon la rev. 1, caractérisé en ce que l'on prépare le méthylène-(2S,5R)-6-[(Z)-acétonylidène]-3,3-diméthyl-7-oxo-4-thia-1-azabicyclo-[3.2.0]heptane-2-carboxylate-pivalate.

7. Procédé selon la rev. 1, caractérisé en ce que l'on prépare le (2S,5R)-6-[(Z)-acétonylidène]-3,3-diméthyl-7-oxo-4-thia-1-azabicyclo[3.2.0]heptane-2-carboxylate de sodium.

8. Procédé selon la rev. 1, caractérisé en ce que l'on prépare le méthylène-(2S,5R)-3,3-diméthyl-6-(3-méthyl-2-oxobutylidène)-7-oxo-4-thia-1-azabicyclo[3.2.0]heptane-2-carboxylate-pivalate.

9. Procédé selon la rev. 1, caractérisé en ce que l'on prépare le méthylène-(2S,5R)-3,3-diméthyl-7-oxo-6-(2-oxo-3-phénoxypropylidène)-4-thia-1-azabicyclo[3.2.0]heptane-2-carboxylate-pivalate.

10. Procédé selon la rev. 1, caractérisé en ce que l'on prépare le méthylène-(2S,5R)-3,3-diméthyl-7-oxo-6-(2-oxo-3-phénylpropylidène)-4-thia-1-azabicyclo[3.2.0]heptane-2-carboxylate-pivalate.

11. Procédé selon la rev. 1, caractérisé en ce que l'on prépare le méthylène-(2S,5R)-3,3-diméthyl-7-oxo-6-(3-methylthio-2-oxopropylidène)-4-thia-1-azabicyclo[3.2.0]heptane-2-carboxylate-pivalate.

12. Procédé selon la rev. 1, caractérisé en ce que l'on prépare le 4,4-dioxyde du méthylène-(2S,5R)-6-acétonylidène-3,3-diméthyl-7-oxo-4-thia-1-azabicyclo[3.2.0]heptane-2-carboxylate-pivalate.

13. Procédé selon la rev. 1, caractérisé en ce que l'on prépare le méthylène-(2S,5R)-3,3-diméthyl-6-(1-chloro-2-oxopropylidène)-7-oxo-4-thia-1-azabicyclo[3.2.0]heptane-2-carboxylate-pivalate.

14. Procédé selon la rev. 1, caractérisé en ce que l'on prépare le méthylène-(2S,5R)-6-(3-chloracétonylidène)-3,3-diméthyl-7-oxo-4-thia-1-azabicyclo[3.2.0]heptane-2-carboxylate-pivalate.

15. Procédé selon la rev. 1, caractérisé en ce que l'on prépare le 4-oxyde du méthylène-(2S,5R)-6-acétonylidène-3,3-diméthyl-7-oxo-4-thia-1-azabicyclo[3.2.0]heptane-2-carboxylate-pivalate.

16. Procédé selon la rev. 1, caractérisé en ce que l'on prépare le (E)- et le (Z)-méthylène-(2S,5R)-6-(formylméthylène)-3,3-diméthyl-7-oxo-4-thia-1-azabicyclo[3.2.0]heptane-2-carboxylate-pivalate.

17. Procédé de préparation des composés de formule générale

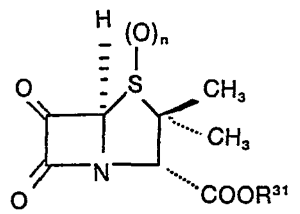

(II)

dans laquelle $R^{31}$ a les mêmes significations que $R^3$ à l'exception de l'hydrogène et n est égal à 0, 1 ou 2, caractérisé en ce que l'on oxyde un composé de formule générale

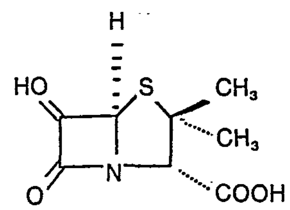

(IV)

dans laqelle $R^{31}$ a la signification indiquée ci-dessus.

18. Procédé selon la rev. 17, pour la préparation d'un composé de formule II dans laquelle n est égal à 0, caractérisé en ce que l'on oxyde le composé de formule V par l'anhydride trifluoracétique et le diméthylsulfoxyde.

19. Procédé selon la rev. 18, caractérisé en ce que l'on convertit le produit obtenu, à l'aide d'un peroxyde, en un composé de formule II dans laquelle n est égal à 1 ou 2.

20. Procédé selon la rev. 19, caractérisé en ce que le peroxyde utilisé est l'acide m-chloroperbenzoïque.